# EUROPEAN PATENT SPECIFICATION

(11) **EP 1 127 135 B1**
(45) Date of publication and mention of the grant of the patent: **23.05.2007**
(21) Application number: 99956776.1
(22) Date of filing: 29.10.1999
(51) Int. Cl.: C12N 15/52, C12N 9/00, C12N 1/21, C12Q 1/68

(54) **HIGH FIDELITY THERMOSTABLE LIGASE AND USES THEREOF**
HIGH FIDELITY THERMOSTABILE LIGASE UND IHRE VERWENDUNG
LIGASE THERMOSTABLE HAUTE FIDELITE ET SES UTILISATIONS

(30) Priority: 30.10.1998 US 106461 P
(43) Date of publication of application: 29.08.2001
(73) Proprietor: CORNELL RESEARCH FOUNDATION, INC., Ithaca, NY 14850 (US)
(72) Inventor: BARANY, Francis, New York, NY 10021 (US); CAO, Weiguo, New York, NY 10021 (US); TONG, Jie, ForestHills, NY 11375 (US)
(74) Representative: Jones, Stephen Anthony
(86) International application number: PCT/US1999/025437
(87) International publication number: WO 2000/026381

(56) References cited:
- WO-A-91/17239
- WO-A-98/03673
- J. TONG ET AL.: "Biochemical properties of a high fidelity DNA ligase from Thermus species AK16D" NUCLEIC ACIDS RESEARCH, vol. 27, no. 3, 1 February 1999 (1999-02-01), pages 788-794, XP002141349 IRL PRESS LIMITED,OXFORD,ENGLAND
- M. ZIRVI ET AL.: "Ligase-based detection of mononucleotide repeat sequences" NUCLEIC ACIDS RESEARCH, vol. 27, no. 24, 15 December 1999 (1999-12-15), page e40 XP002141350 IRL PRESS LIMITED,OXFORD,ENGLAND
- J. LUO AND F. BARANY: "Identification of essential residues in Thermus thermosphilus DNA ligase" NUCLEIC ACIDS RESEARCH, vol. 24, no. 15, 1 August 1996 (1996-08-01), pages 3079-3085, XP002141351 IRL PRESS LIMITED,OXFORD,ENGLAND cited in the application
- J. LUO ET AL.: "Improving the fidelity of Thermus thermophilus DNA ligase" NUCLEIC ACIDS RESEARCH, vol. 24, no. 14, 1 August 1996 (1996-08-01), pages 3071-3078, XP002141352 IRL PRESS LIMITED,OXFORD,ENGLAND cited in the application
- BARANY F: "GENETIC DISEASE DETECTION AND DNA AMPLIFICATION USING CLONED THERMOSTABLE LIGASE" PROCEEDINGS OF THE NATIONAL ACADEMY OF SCIENCES OF USA,US,NATIONAL ACADEMY OF SCIENCE. WASHINGTON, vol. 88, no. 1, 1 January 1991 (1991-01-01), pages 189-193, XP000368693 ISSN: 0027-8424 cited in the application
- JONSSON Z O ET AL: "Sequence of the DNA ligase-encoding gene from Thermus scotoductus and conserved motifs in DNA ligases" GENE,NL,ELSEVIER BIOMEDICAL PRESS. AMSTERDAM, vol. 151, no. 1, 30 December 1994 (1994-12-30), pages 177-180, XP004042633 ISSN: 0378-1119 cited in the application
- F. BARANY AND D.H. GELFAND: "Cloning, overexpression and nucleotide sequence of a thermostable DNA ligase-encoding gene" GENE, vol. 109, no. 1, 20 December 1991 (1991-12-20), pages 1-11, XP002141353 ELSEVIER SCIENCE PUBLISHERS,B.V.,AMSTERDAM,NL;

## Description

This application claims benefit of U.S. Provisional Patent Application Serial No. 60/106,461 October 30, 1998.

The present invention was made with support under National Institutes of Health Grant Nos. GM-41337-09 and PO1-CA65930-02-04). The U.S. Government may have certain rights.

### FIELD OF THE INVENTION

The present invention is directed to a high fidelity thermostable ligase and uses thereof.

### BACKGROUND OF THE INVENTION

DNA ligases, as an essential component of DNA replication, recombination, and repair systems found from viruses to humans, catalyze the formation of a phosphodiester bond at single-stranded breaks on duplex DNA (Lehman, I.R., Science, 186:790-797 (1974)). DNA ligases can be classified into two families based on cofactor dependence. ATP-dependent ligases are found in bacteriophages (Dunn, et al., J Mol Biol., 148(4):303-330 (1981) and Weiss, et al., Proc Natl Acad Sci USA, 57(4):1021-1028 (1967)), *Chlorella* virus PBCV-1 (Ho, et al., J Virol, 71 (3):1931-19374 (1997)), Vaccinia virus (Shuman, S., Biochemistry, 34(49):16138-161475 (1995)), Archea (Kletzin, A., Nucleic Acids Res, 20(20):5389-5396 (1992) and Bult, et al., Science, 273(5278):1058-1073 (1996)), yeasts (Andaluz, et al., Yeast, 12(9):893-8988 (1996), Ramos, et al., Nucleic Acids Res, 25(8):1485-1492 (1997), Schar, et al., Genes Dev, 11(15):1912-1924 (1997)), mammalian (Tomkinson, et al., Bioessays, 19(10):893-901 (1997), Tomkinson, et al., Mutat Res, 407(1):1-9 (1998), and Wang, et al., J Biol Chem, 269(50):31923-3192811 (1994)), and more recently eubacteria (Cheng, et al., Nucleic Acids Res, 25(7):1369-1374 (1997) and Deckert, et al., Nature, 392(6674):353-358 (1998)). NAD⁺(i.e. nicotinamide adenine dinucleotide)-dependent ligases, however, are found exclusively in eubacteria. While some higher eucaryotic organisms may use multiple ATP (i.e. adenosine triphosphate)-dependent ligases to fulfill diverse biological functions, some simple eubacteria genomes could host both an NAD⁺-dependent ligase and an ATP-dependent ligase (Deckert, et al., Nature, 392(6674):353-358 (1998) and Fleischmann, et al., Science, 269(5223):496-512 (1995)). The origin of the additional ATP-dependent ligases in these genomes remains to be determined.

Although the ATP-dependent ligases and NAD⁺-dependent ligases share little sequence homology, all the ligases investigated so far use the same KXDG motif to form adenylated enzyme intermediate (Tomkinson, et al., Bioessays, 19(10):893-901 (1997), Shuman, et al., Virology, 211(1):73-83 (1995), and Luo, et al., Nucleic Acids Res, 24(15):3079-3085 (1996)). Furthermore, they seem to be organized by similar domains and structural folds ((Doherty, et al., Nucleic Acids Res, 24(12):2281-2287 (1996), Subramanya, et al., Cell, 85(4):607-615 (1996), and Sekiguchi, et al., Nucleic Acids Res, 25(4):727-734 (1997)). The diversity of ligase sequences is not only reflected by their different optimal reaction conditions and kinetic rates, but more importantly by their different specificities toward match and mismatch substrates. Among the viral ATP-dependent ligases, the broad substrate tolerance is represented by the T4 enzyme which seals various mismatches on both the 3' and 5' side of the nick junction (Wu, et al., Gene, 76(2):245-254 (1989)). Vaccinia ligase ligates various mismatches at both 3'-hydroxyl or 5'-phosphate sides with the exception of purine-purine mismatch pairs at the 3'-hydroxyl side (Shuman, S., Biochemistry, 34(49):16138-161475 (1995)). Mammalian ATP-dependent ligases show different substrate sensitivity, as ligase I is more sensitive to 3' mismatches than ligase III (Husain, et al., J Biol Chem, 270(16):9683-9690 (1995)). Additionally, both ligase I and III tolerate a 3'C/T mismatch more than a 3'G/T mismatch. Little is known about archeal ATP-dependent ligases which may reveal the nature of the progenitor of ATP-dependent ligases. Studies on NAD⁺-dependent DNA ligase from *E. coli,* along with T4 ligase, have contributed immensely to understanding of the basic biochemical pathway of the DNA ligation reaction (Lehman, I.R., Science, 186(4166):790-797 (1974) and Rossi, et al., Nucleic Acids Res, 25(11):2106-2113 (1997)). Studies on the NAD⁺-dependent ligase from *Thermus thermophilus* HB8 have revealed the highly discriminative power this enzyme possesses (Luo, et al., Nucleic Acids Res, 24(15):3071-3078 (1996)). Although mismatches at 5'-phosphate side are tolerated to some degree (5'A/C, 5'A/A, 5'C/A, 5'C/T, 5'G/T, 5'G/A, 5'T/T, 5'T/G), mismatches at the 3'-hydroxyl side essentially abolish nick-closure activity except 3'G/T or 3'T/G mismatch (Luo, et al., Nucleic Acids Res, 24(15):3071-3078 (1996)). Apparently, sequence divergence and subsequent subtle structural variation among DNA ligases underlie an enzyme's recognition preferences toward different mismatched base-pairs.

The study of ligase biochemistry is not only important for understanding its biological functions, but also for developing new technologies. The single nucleotide discrimination observed on DNA ligases has led to the development of ligase-mediated detection techniques (Wu, et al., Gene, 76(2):245-254 (1989), Wu, et al., Genomics, 4(4):560-569 (1989), Landegren, et al., Science, 241(4869):1077-1080 (1988), Landegren, U., Bioessays, 15(11):761-765 (1993), Barany, F., PCR Methods Appl, 1(1):5-16 (1991), and Barany, F., Proc Natl Acad Sci USA, 88(1):189-193 (1991)). Ligase-based linear signal amplification known as LDR (i.e. ligase detection reaction), combined with PCR (i.e. polymerase chain reaction)-based gene specific target amplification, has been proven to be a powerful tool in cancer and disease gene mutation detection (Day, et al., Genomics, 29(1):152-162 (1995)). PCR/LDR technique relies on two properties of a DNA ligase: (i) specificity and (ii) thermostability. *Tth* (i.e. *Thermus thermophilus* HB8) DNA ligase has been successfully used in LDR and LCR (i.e. ligase chain reaction) due to its highly discriminative nick closure activity toward a perfect match substrate and its thermostability which makes thermocycling possible (Barany, F., PCR Methods Appl, 1(1):5-16 (1991) and Barany, F., Proc Natl Acad Sci USA, 88(1):189-193 (1991)). To date, one more ligase was cloned and sequenced from T. Scot. (i.e. *Thermus scotoductus)* (Thorbjarnardottir, et al., Gene, 161(1):1-6 (1995) and Jonsson, et al., Gene, 151(1-2):177-180 (1994)), but the substrate specificity of this ligase was not determined.

Despite the existence of a number of ligases from different host sources, the need remains to identify additional ligases with greater fidelity. The present invention is directed to achieving this objective as a result of the cloning and expression of a ligase from T. sp. (i.e. *Thermus* species) AK16D and the biochemical characterization of this high fidelity enzyme.

### SUMMARY OF THE INVENTION

The present invention is directed to an isolated thermostable ligase as defined in independent Claim 1.

The present invention also relates to a DNA molecule encoding the thermostable ligase as well as expression systems and host cells containing such DNA molecules.

Another aspect of the present invention relates to the use of the thermostable ligase in carrying out a ligase detection reaction process or a ligase chain reaction process.

The ligase detection reaction process, involves detecting a target nucleotide sequence which differs from other nucleotide sequences in the sample by one or more single base changes, insertions, deletions, or translocations. This involves providing a sample potentially containing a target nucleotide sequence which differs from other nucleotide sequences in the sample by one or more single base changes, insertions, deletions, or translocations.

The method further includes providing one or more oligonucleotide probe sets, each characterized by (a) a first oligonucleotide probe having a target specific portion and (b) a second oligonucleotide probe having a target-specific portion. The oligonucleotide probes in a particular set are suitable for hybridization to a target nucleotide sequence which differs from other nucleotide sequences in the sample by one or more single base changes, insertions, deletions, or translocations. The probes are also suitable for ligation together when hybridized adjacent to one another on the target nucleotide sequence, but have a mismatch which interferes with such ligation when hybridized to any other nucleotide sequence present in the sample.

The sample, the one or more oligonucleotide probe sets, and the thermostable ligase are blended to form a ligase detection reaction mixture. The ligase detection reaction mixture is subjected to one or more ligase detection reaction cycles comprising a denaturation treatment and a hybridization treatment. In the denaturation treatment, any hybridized oligonucleotides are separated from the target nucleotide sequence. During the hybridization treatment, the oligonucleotide probe sets hybridize at adjacent positions in a base specific manner to their respective target nucleotide sequences, if present in the sample, and ligate to one another. This forms a ligation product sequence containing the target specific portions connected together with the ligation product sequences for each set being distinguishable from other nucleic acids in the ligase detection reaction mixture. The oligonucleotide probe sets may hybridize to a nucleotide sequence in the sample other than their respective target nucleotide sequences but do not ligate together due to a presence of one or more mismatches and individually separate during the denaturation treatment. The presence of ligation product sequences produced as a result of the target nucleotide sequence being present in the sample is then detected.

In the ligase chain reaction process of the present invention, the presence of a target double stranded nucleic acid formed from first and second complementary target nucleotide sequences is detected in a sample. The target double stranded nucleic acid differs from other nucleotide sequences by one or more single base changes, insertions, deletions, or translocations.

This method involves providing a sample potentially containing a target double stranded nucleic acid formed from first and second complementary nucleotide sequence. This nucleic acid differs from other nucleotide sequences in the sample by one or more single base changes, insertions, deletions, or translocations.

The method further includes providing a first oligonucleotide probe set, characterized by (a) a first oligonucleotide probe having a target specific portion and (b) a second oligonucleotide probe having a target-specific portion. The oligonucleotide probes in the first set are complementary to the first target nucleotide sequence which differs from other nucleotide sequences in the sample by one or more single base changes, insertions, deletions, or translocations. The probes are also suitable for ligation together when hybridized adjacent to one another on the first target nucleotide sequence, but have a mismatch which interferes with such ligation when hybridized to any other nucleotide sequence present in the sample. The method of the present invention also requires providing a second oligonucleotide probe set, characterized by (a) a third oligonucleotide probe having a target specific portion and (b) a fourth oligonucleotide probe having a target-specific portion. The oligonucleotide probes in the second set are complementary to the second target nucleotide sequence which differs from other nucleotide sequences in the sample by one or more single base changes, insertions, deletions, or translocations. The probes of the second set are suitable for ligation together when hybridized adjacent to one another on the second target nucleotide sequence, but have a mismatch which interferes with such ligation when hybridized to any other nucleotide sequence present in the sample.

The sample, the first and second oligonucleotide probe sets, and the thermostable ligase are blended together to form a ligase chain reaction mixture. The ligase chain reaction mixture is subjected to one or more ligase chain reaction cycles comprising a denaturation treatment and a hybridization treatment. During the denaturation treatment, any hybridized oligonucleotides are separated from the target nucleotide sequences. In the hybridization treatment, the oligonucleotide probe sets hybridize at adjacent positions in a base specific manner to their respective target nucleotide sequences, if present in the sample. The probes also ligate to one another to form a ligation product sequence containing the target specific portions connected together with the ligation product sequences for each set being distinguishable from other nucleic acids in the ligase chain reaction mixture. The oligonucleotide probe sets may hybridize to nucleotide sequences in the sample other than their respective target nucleotide sequences but do not ligate together due to a presence of one or more mismatches and individually separate during the denaturation treatment. The presence of ligation product sequences produced as a result of the target nucleotide sequence being present in the sample are then detected.

### BRIEF DESCRIPTION OF THE DRAWINGS

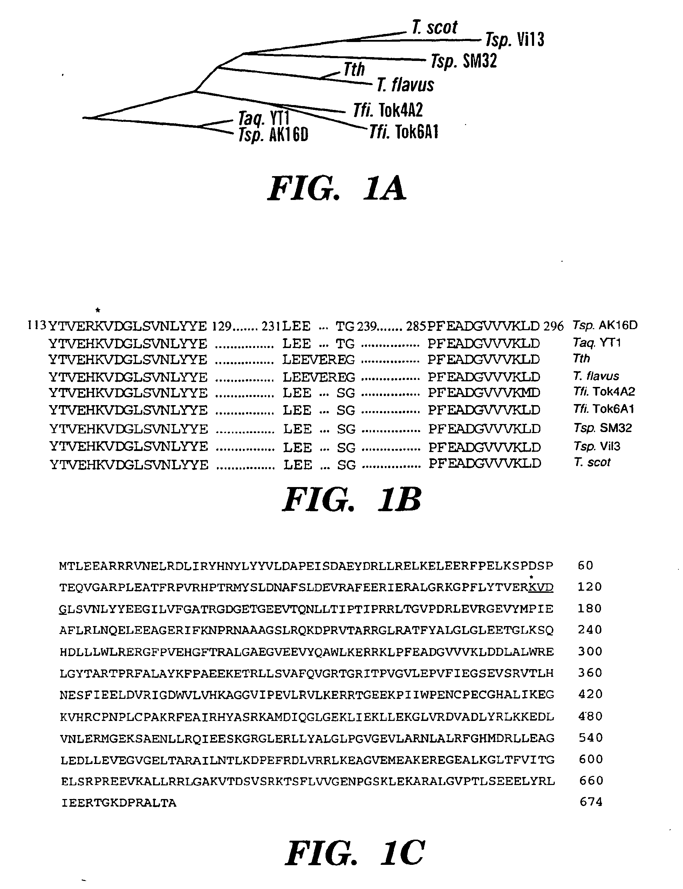

Figures 1A-C show a sequence comparison of *Thermus* DNA ligases. Figure 1A illustrates the evolutionary tree for *Thermus* DNA ligases. Figure 1B is a regional sequence alignment of nine *Thermus* ligases. The aa (i.e. amino acid) sequence of T. scot is retrieved from Genebank by accession number 1085749. The adenylation motif KXDG is underlined and the adenylation site is marked by *. The numbering of aa is based on *Tsp.* AK16D ligase. Figure 1C is a complete amino acid sequence of *Tsp.* AK16D ligase. The adenylation motif KXDG is underlined and the adenylation site ¹¹⁸K is shown with a (*) above the residue. The complete sequence of *Tsp.* AK16D ligase gene and partial sequences of six other *Thermus* ligase genes have been deposited with GenBank under accession No. AF092862 for *Tsp.* AK16D, AF092863 for *Thermus aquaticus* YT-1, AF092864 for *Thermus flavus,* AF092865 for *Thermus filiformis* Tok4A2, AF092866 for *Thermus filiformis* Tok6Al, AF092867 for *Tsp.* Vil3, and AF092868 for *Tsp.* SM32.

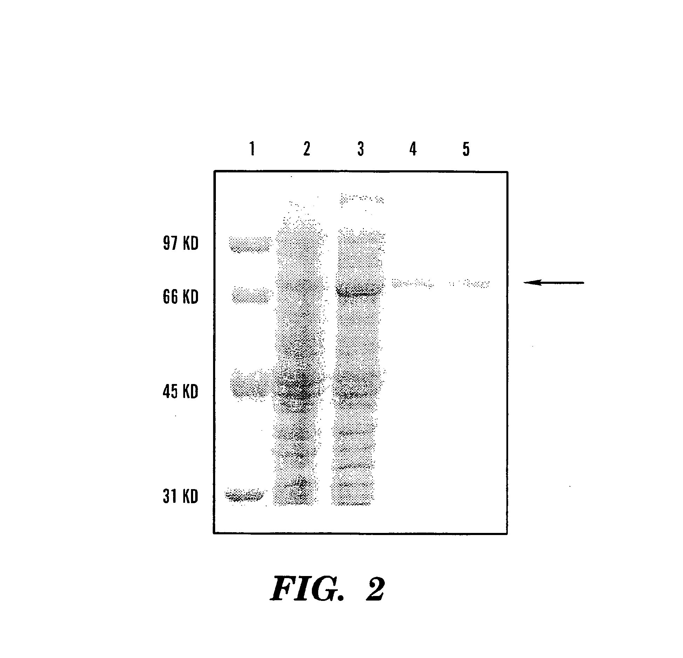

Figure 2 shows an SDS-PAGE analysis of *Tsp.* AK16D ligase protein. Lane 1, molecular weight markers; Lane 2, uninduced cell lysate; Lane 3, induced cell lysate; Lane 4, supernatant after heating at 70°C; Lane 5, fraction eluted from Hitrap blue column. The SDS-polyacrylamide gel was 0.1% SDS-7.5% polyacrylamide and was stained with Coomassie brilliant blue after electrophoresis. The arrow points to the location of *Tsp.* AK16D ligase.

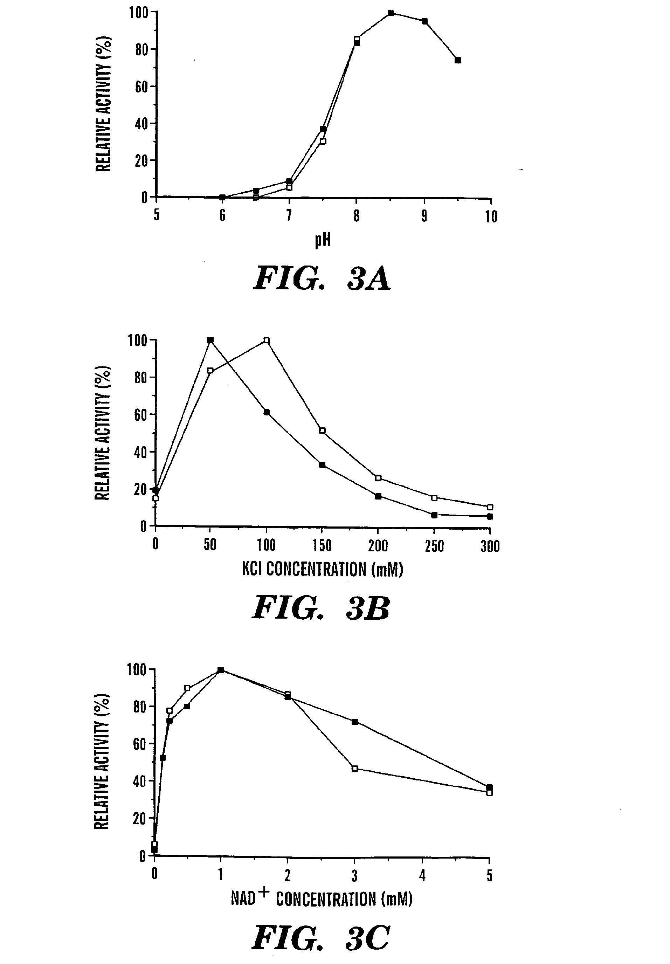

Figures 3A-C show the effects of salt, pH, and NAD⁺ on ligation activity. *Tsp.* AK16D ligase: closed squares; *Tth* ligase: open squares. Figure 3A reveals the pH effect. Reactions were performed in 20 µl mixture containing 200 nM nicked duplex substrate, 12.5 pM *Tth* ligase or *Tsp.* AK16D ligase, 20 mM Tris-HCl (pH values were determined at room temperature), 10 mM MgCl₂, 100 mM KCl, 10 mM DTT, 1 mM NAD⁺ and 20 mg/ml BSA at 65°C for 10 min. Figure 3B shows the salt effect. Reactions were performed in 20 µl mixture containing 200 nM nicked duplex substrate, 12.5 pM *Tth* ligase or *Tsp.* AK16D ligase, 20 mM Tris-HCl, pH 8.5 (at room temperature) for *Tth* ligase, pH 8.0 for *Tsp.* AK16D ligase, 10 mM MgCl₂, indicated amount of KCl, 10 mM DTT, 1 mM NAD⁺ and 20 mg/ml BSA at 65°C for 10 min. Figure 3C shows the NAD⁺ effect. *Tth* ligation reactions were performed in 20 µl mixture containing 200 nM nicked duplex substrate, 12.5 pM *Tth* ligase and indicated concentration of NAD⁺, 20 mM Tris-HCl, pH 8.5, 5 mM MgCl₂, 100 mM KCl, 10 mM DTT, 1 mM NAD⁺ and 20 mg/ml BSA at 65°C for 10 min. *Tsp.* AK16D ligation reaction were performed in 20 µl mixture containing 200 nM nicked duplex substrate, 12.5 pM *Tth* ligase and indicated concentration of NAD⁺, 20 mM Tris-HCl, pH 8.5, 5 mM MgCl₂, 50 mM KCl, 10 mM DTT, 1 mM NAD⁺ and 20 mg/ml BSA at 65°C for 10 min.

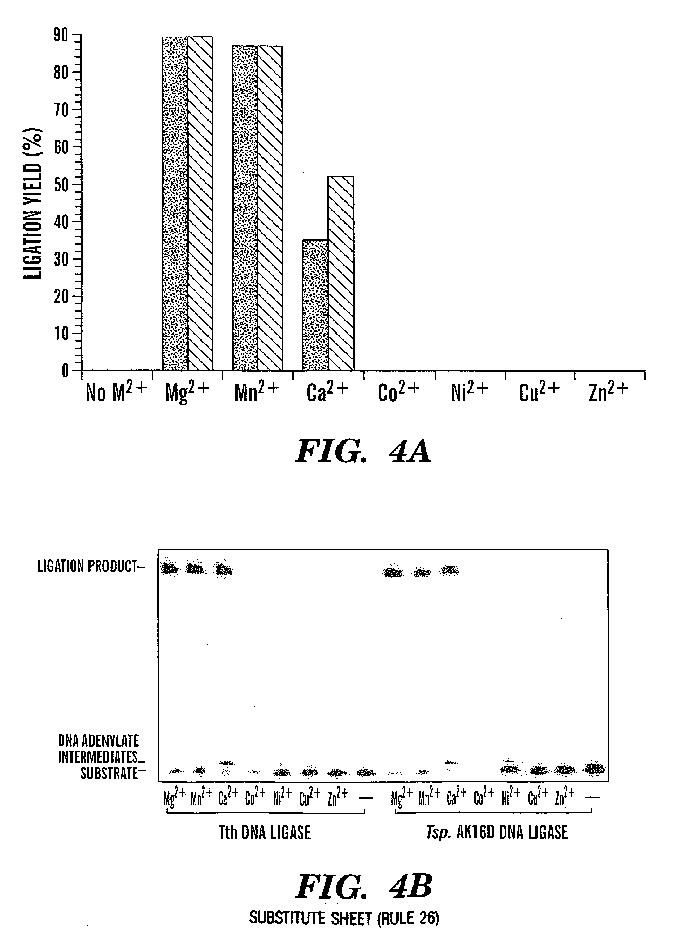

Figures 4A-B show the divalent cation dependence of *Tsp.* AK16D (stripped bars) and *Tth* (filled bars) ligase activity. Reaction mixtures containing (20 µl) 20 nM nicked duplex substrate, 0.5 nM *Tth* ligase or 1 nM *Tsp.* AK16D ligase and 5 mM of indicated divalent cation in the reaction buffers as specified in Figure 3C were incubated at 65°C for 10 min. Figure 4A shows the ligation reactions with different divalent ions as the metal cofactor. Figure 4B shows the chromatogram of a representative GeneScan gel illustrating ligation product and DNA adenylate intermediate. (-): negative control reactions in which ligase was omitted. Co²⁺ may have caused precipitation of DNA substrate which resulted in disappearance of the unreacted substrate.

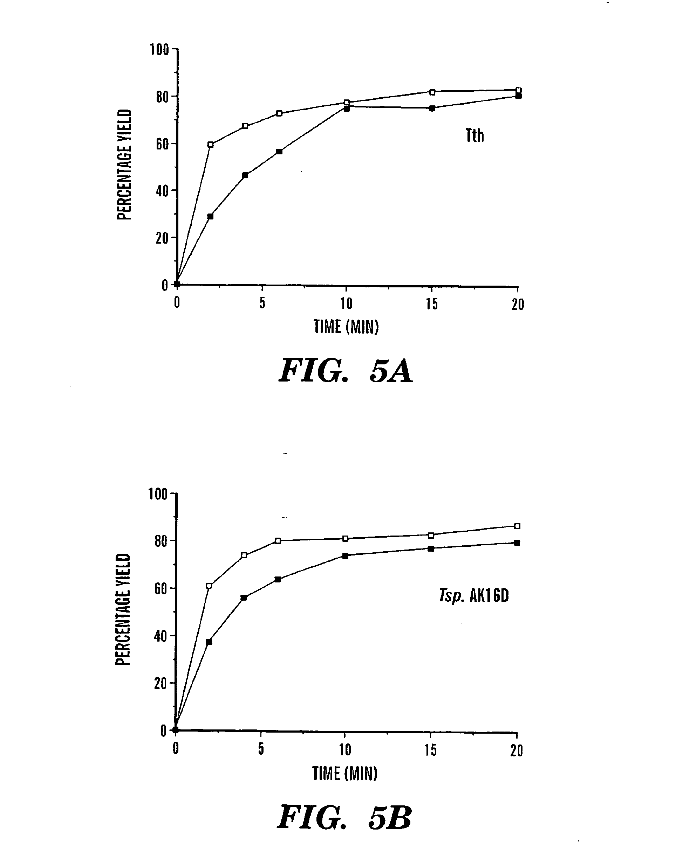

Figures 5A-B shows the time course of *Tth* (Figure 5A) and *Tsp.* AK16D (Figure 5B) ligase activity in the presence of Mg²⁺ (open squares) or Mn²⁺ (closed squares). Reactions were performed in 100 µl mixture containing 20 nM nicked duplex substrate, 0.5 nM *Tth* ligase or 1 nM *Tsp.* AK16D ligase and 5 mM Mg²⁺ or Mn²⁺ in the reaction buffers as specified in Figure 3C at 65°C. Aliquots (5 µl) were removed at the indicated time and reactions stopped by adding equal volumes of stop solution.

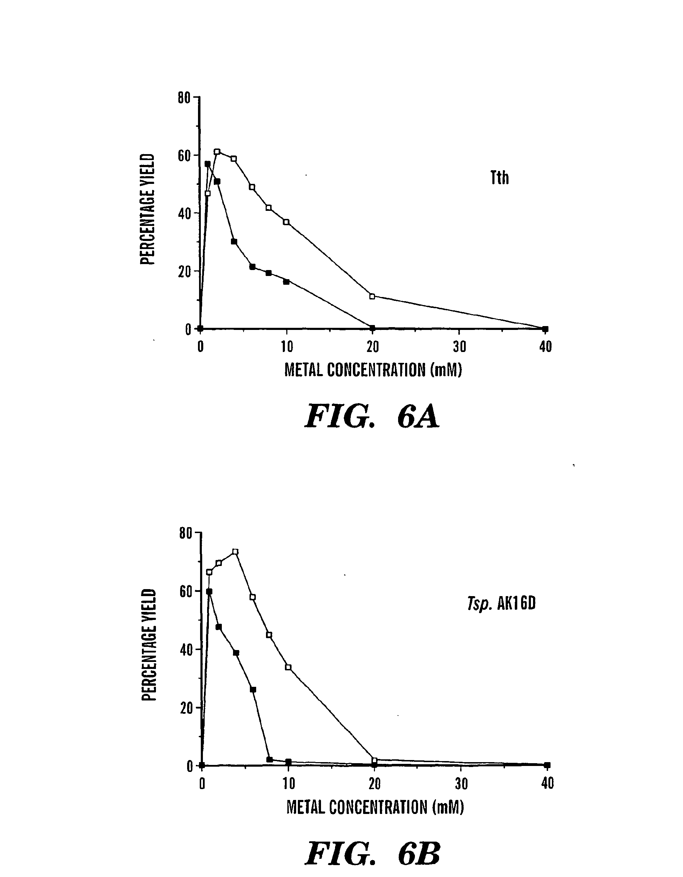

Figures 6A-B show the divalent cation concentration dependence of *Tth* (Figure 6A) and *Tsp.* AK16D (Figure 6B) ligase activity. Mg²⁺(open squares); Mn²⁺ (closed squares). Reactions were performed in 20 µl mixture containing 20 nM nicked duplex substrate, 0.5 nM *Tth* ligase or 1 nM *Tsp.* AK16D ligase and indicated concentration of Mg²⁺ or Mn²⁺ in the reaction buffers as specified in Figure 4C at 65°C for 2 min.

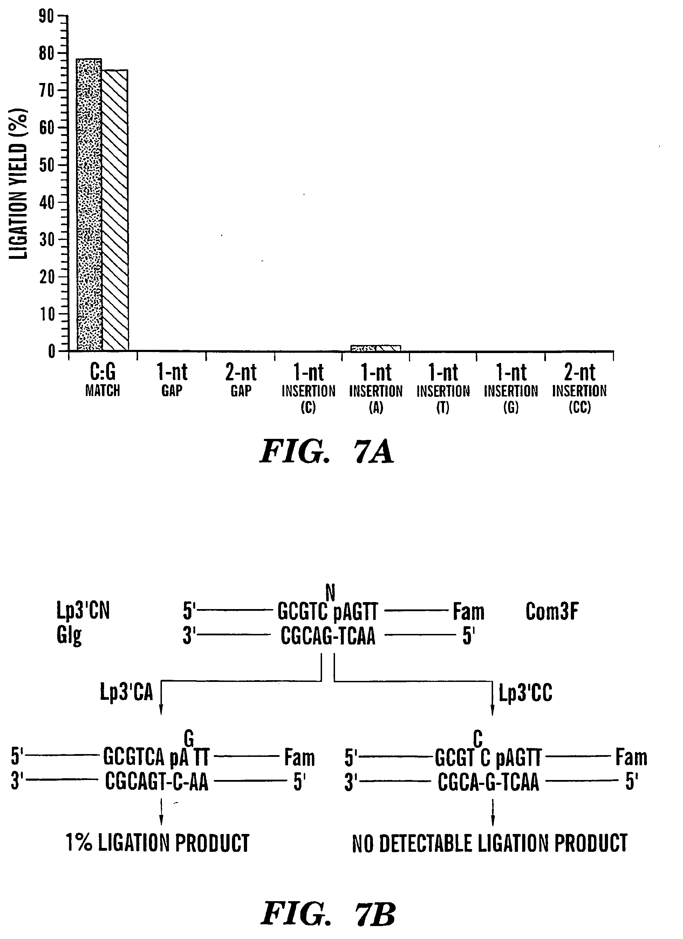

Figures 7A-B show the ligation of gapped and inserted substrates. Figure 7A shows the formation of ligated product with gapped and inserted substrates. Reactions were performed in a 20 µl mixture containing 12.5 nM nicked duplex substrate, 1.25 pM *Tth* ligase or 12.5 nM *Tsp.* AK16D ligase in the reaction buffer at 65°C for 4 hours. Figure 7B shows the proposed reaction path leads to ligation of 1 nt (i.e. nucleotides) inserted substrate.

### DETAILED DESCRIPTION OF THE INVENTION

The present invention relates to a high fidelity thermostable ligase enzyme. This enzyme has the amino acid sequence of SEQ. ID. No. 1 as follows:

This protein has a molecular weight of 78 to 81 kDa, as measured by SDS-PAGE. For purposes of the present application, the term "thermostable" refers to a DNA ligase which is resistant to inactivation by heat.

The thermostable ligase of the present invention has a 100 fold higher fidelity than T4 ligase and 6 fold higher fidelity than wild-type *Thermus thermophilus* ligase, when sealing a ligation junction between a pair of oligonucleotide probes hybridized to a target sequence where there is a mismatch with the oligonucleotide probe having its 3' end abutting the ligation junction at the base immediately adjacent the ligation junction. This ligase also has a 50 fold higher fidelity than T4 ligase and 5 fold higher fidelity than wild-type *Thermus thermophilus* ligase, when sealing a ligation junction between a pair of oligonucleotide probes hybridized to a target sequence where there is a mismatch with the oligonucleotide probe having its 3' end abutting the ligation junction at the base penultimate to the ligation junction. Finally, the thermostable ligase of the present invention, in the presence of a Mn²⁺ cofactor, has a 12 fold higher fidelity than wild-type *Thermus thermophilus* ligase, when sealing a ligation junction between a pair of oligonucleotide probes hybridized to a target sequence where there is a mismatch with the oligonucleotide probe having its 3' end abutting the ligation junction at the base immediately adjacent to the ligation junction. For purposes of the present invention, "fidelity" is defined to mean the ratio of the initial rate of ligating two adjacent probes hybridized to a complementary template with a C-G match at the base of the probe with its 3' end at the ligation junction to the initial rate of ligating two adjacent probes hybridized to a complementary template with a G-T mismatch at the base of the probe with its 3' end at the ligation junction.

The thermostable ligase of the present invention is also characterized by having an arginine adjacent to the active site lysine (i.e. K) in the KXDG motif (where X is any amino acid).

This protein is encoded by a DNA molecule having a nucleotide sequence of SEQ. ID. No. 2 as follows:

Fragments of the above polypeptide or protein are also encompassed by the present invention.

Suitable fragments can be produced by several means. In the first, subclones of the gene encoding the protein of the present invention are produced by conventional molecular genetic manipulation by subcloning gene fragments. The subclones then are expressed *in vitro* or *in vivo* in bacterial cells to yield a smaller protein or peptide that can be tested for ligase activity according to the procedure described below.

As an alternative, fragments of the ligase of the present invention can be produced by digestion of the full-length ligase with proteolytic enzymes like chymotrypsin or *Staphylococcus* proteinase A, or trypsin. Different proteolytic enzymes are likely to cleave ligase proteins at different sites based on the amino acid sequence of the ligase. Some of the fragments that result from proteolysis may be active ligases.

In another approach, based on knowledge of the primary structure of the protein, fragments of the ligase encoding gene may be synthesized by using the PCR technique together with specific sets of primers chosen to represent particular portions of the protein. These then would be cloned into an appropriate vector for increased expression of a truncated peptide or protein.

Chemical synthesis can also be used to make suitable fragments. Such a synthesis is carried out using known amino acid sequences for the ligase being produced. Alternatively, subjecting the full length ligase to high temperatures and pressures will produce fragments. These fragments can then be separated by conventional procedures (e.g., chromatography, SDS-PAGE).

Variants may also (or alternatively) be modified by, for example, the deletion or addition of amino acids that have minimal influence on the properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification, or identification of the polypeptide.

Suitable DNA molecules are those that hybridize to a DNA molecule comprising a nucleotide sequence of 50 continuous bases of SEQ. ID. No. 2 under stringent conditions characterized by a hybridization buffer comprising 0.9M sodium citrate ("SSC") buffer at a temperature of 37°C and remaining bound when subject to washing with the SSC buffer at 37°C; and preferably in a hybridization buffer comprising 20% formamide in 0.9M saline/0.09M SSC buffer at a temperature of 42°C and remaining bound when subject to washing at 42°C with 0.2x SSC buffer at 42°C.

The protein or polypeptide of the present invention is preferably produced in purified form (preferably at least about 80%, more preferably 90%, pure) by conventional techniques. Typically, the protein or polypeptide of the present invention is secreted into the growth medium of recombinant host cells.
Alternatively, the protein or polypeptide of the present invention is produced but not secreted into growth medium. In such cases, to isolate the protein, the host cell (e.g., *E. coli*) carrying a recombinant plasmid is propagated, lysed by sonication, heat, or chemical treatment, and the homogenate is centrifuged to remove bacterial debris. The supernatant is then subjected to sequential ammonium sulfate precipitation. The fraction containing the polypeptide or protein of the present invention is subjected to gel filtration in an appropriately sized dextran or polyacrylamide column to separate the proteins. If necessary, the protein fraction may be further purified by HPLC.

The DNA molecule encoding the ligase of the present invention can be incorporated in cells using conventional recombinant DNA technology. Generally, this involves inserting the DNA molecule into an expression system to which the DNA molecule is heterologous (i.e. not normally present). The heterologous DNA molecule is inserted into the expression system or vector in proper sense orientation and correct reading frame. The vector contains the necessary elements for the transcription and translation of the inserted protein-coding sequences.

U.S. Patent No. 4,237,224 to Cohen and Boyer describes the production of expression systems in the form of recombinant plasmids using restriction enzyme cleavage and ligation with DNA ligase. These recombinant plasmids are then introduced by means of transformation and replicated in unicellular cultures including procaryotic organisms and eucaryotic cells grown in tissue culture.

Recombinant genes may also be introduced into viruses, such as vaccina virus. Recombinant viruses can be generated by transfection of plasmids into cells infected with virus.

Suitable vectors include, but are not limited to, the following viral vectors such as lambda vector system gt11, gt WES.tB, Charon 4, and plasmid vectors such as pBR322, pBR325, pACYC177, pACYC1084, pUC8, pUC9, pUC18, pUC19, pLG339, pR290, pKC37, pKC101, SV 40, pBluescript II SK +/- or KS +/- (see "Stratagene Cloning Systems" Catalog (1993) from Stratagene, La Jolla, Calif , pQE, pIH821, pGEX, pET series (see F.W. Studier et. al., "Use of T7 RNA Polymerase to Direct Expression of Cloned Genes," Gene Expression Technology vol. 185 (1990), which is hereby incorporated by reference), and any derivatives thereof. Recombinant molecules can be introduced into cells via transformation, particularly transduction, conjugation, mobilization, or electroporation. The DNA sequences are cloned into the vector using standard cloning procedures in the art, as described by Sambrook et al., Molecular Cloning: A Laboratory Manual, Cold Springs Laboratory, Cold Springs Harbor, New York (1989).

A variety of host-vector systems may be utilized to express the protein-encoding sequence(s). Primarily, the vector system must be compatible with the host cell used. Host-vector systems include but are not limited to the following: bacteria transformed with bacteriophage DNA, plasmid DNA, or cosmid DNA; microorganisms such as yeast containing yeast vectors; mammalian cell systems infected with virus (e.g., vaccinia virus, adenovirus, etc.); insect cell systems infected with virus (e.g., baculovirus); and plant cells infected by bacteria. The expression elements of these vectors vary in their strength and specificities. Depending upon the host-vector system utilized, any one of a number of suitable transcription and translation elements can be used.

Different genetic signals and processing events control many levels of gene expression (e.g., DNA transcription and messenger RNA (mRNA) translation).

Transcription of DNA is dependent upon the presence of a promotor which is a DNA sequence that directs the binding of RNA polymerase and thereby promotes mRNA synthesis. The DNA sequences of eucaryotic promotors differ from those of procaryotic promotors. Furthermore, eucaryotic promotors and accompanying genetic signals may not be recognized in or may not function in a procaryotic system, and, further, procaryotic promotors are not recognized and do not function in eucaryotic cells.

Similarly, translation of mRNA in procaryotes depends upon the presence of the proper procaryotic signals which differ from those of eucaryotes. Efficient translation of mRNA in procaryotes requires a ribosome binding site called the Shine-Daigarno ("SD") sequence on the mRNA. This sequence is a short nucleotide sequence of mRNA that is located before the start codon, usually AUG, which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, Methods in Enzymology, 68:473 (1979),

Promotors vary in their "strength" (i.e. their ability to promote transcription). For the purposes of expressing a cloned gene, it is desirable to use strong promotors in order to obtain a high level of transcription and, hence, expression of the gene. Depending upon the host cell system utilized, any one of a number of suitable promotors may be used. For instance, when cloning in *E. coli,* its bacteriophages, or plasmids, promotors such as the T7 phage promoter, *lac* promotor, *trp* promotor, *recA* promotor, ribosomal RNA promotor, the P_{R} and P_{L} promotors of coliphage lambda and others, including but not limited, to *lac*UV5, *omp*F*, bla, lpp,* and the like, may be used to direct high levels of transcription of adjacent DNA segments. Additionally, a hybrid *irp-lac*UV5 *(tac)* promotor or other *E. coli* promotors produced by recombinant DNA or other synthetic DNA techniques may be used to provide for transcription of the inserted gene.

Bacterial host cell strains and expression vectors may be chosen which inhibit the action of the promotor unless specifically induced. In certain operations, the addition of specific inducers is necessary for efficient transcription of the inserted DNA. For example, the *lac* operon is induced by the addition of lactose or IPTG (isopropylthio-beta-D-galactoside). A variety of other operons, such as *trp, pro,* etc., are under different controls.

Specific initiation signals are also required for efficient gene transcription and translation in procaryotic cells. These transcription and translation initiation signals may vary in "strength" as measured by the quantity of gene specific messenger RNA and protein synthesized, respectively. The DNA expression vector, which contains a promotor, may also contain any combination of various "strong" transcription and/or translation initiation signals. For instance, efficient translation in *E. coli* requires an SD sequence about 7-9 bases 5' to the initiation codon ("ATG") to provide a ribosome binding site. Thus, any SD-ATG combination that can be utilized by host cell ribosomes may be employed. Such combinations include but are not limited to the SD-ATG combination from the *cro* gene or the *N* gene of coliphage lambda, or from the *E. coli* tryptophan E. D, C, B or A genes. Additionally, any SD-ATG combination produced by recombinant DNA or other techniques involving incorporation of synthetic nucleotides may be used.

Once the isolated DNA molecule encoding the ligase of the present invention has been cloned into an expression system, it is ready to be incorporated into a host cell. Such incorporation can be carried out by the various forms of transformation noted above, depending upon the vector/host cell system. Suitable host cells include, but are not limited to, bacteria, virus, yeast, mammalian cells, insect, plant, and the like.

The present invention is useful in a number of processes where a ligase enzyme is conventionally utilized at high temperatures. Generally, these procedures include the ligase detection reaction and the ligase chain reaction.

Both of the ligase detection reaction and ligase chain reaction involve detection of a target sequence and amplification of that sequence at elevated temperatures. In carrying out these procedures, the enzyme is subjected to elevated temperatures but is not degraded due to its thermostable character. The ligase detection reaction and ligase chain reaction procedures are generally described in WO 90/17239 to Barany et. al., F. Barany, et. al., "Cloning, Overexpression, and Nucleotide Sequence of a Thermostable DNA Ligase-Encoding Gene," Gene 109:1-11 (1991), and F. Barany, et. al., "Genetic Disease Detection and DNA Amplification Using Cloned Thermostable Ligase," Proc. Nat'l Acad. Sci. USA 88: 189-93.

The ligase detection reaction process is useful in detecting in a sample a target nucleotide sequence as described more fully below.

One or more oligonucleotide probe sets are provided for use in conjunction with this method. Each set includes (a) a first oligonucleotide probe having a target-specific portion and (b) a second oligonucleotide probe having a target-specific portion. The oligonucleotide probes in a particular set are suitable for ligation together when hybridized adjacent to one another on a corresponding target nucleotide sequence, but have a mismatch which interferes with such ligation when hybridized to any other nucleotide sequence present in the sample.

The sample, the one or more oligonucleotide probe sets, and the ligase are blended to form a ligase detection reaction mixture. The ligase detection reaction mixture is subjected to one or more ligase detection reaction cycles comprising a denaturation treatment and a hybridization treatment. In the denaturation treatment, any hybridized oligonucleotides are separated from the target nucleotide sequences. The hybridization treatment involves hybridizing the oligonucleotide probe sets at adjacent positions in a base-specific manner to the respective target nucleotide sequences, if present in the sample. The hybridized oligonucleotide probes from each set ligate to one another to form a ligation product sequence containing the target-specific portions connected together. The ligation product sequence for each set is distinguishable from other nucleic acids in the ligase detection reaction mixture. The oligonucleotide probe sets may hybridize to adjacent sequences in the sample other than the respective target nucleotide sequences but do not ligate together due to the presence of one or more mismatches. When hydridized oligonucleotide probes do not ligate, they individually separate during the denaturation treatment.

During the ligase detection reaction phase, the denaturation treatment is carried out at a temperature of 80-105°C, while hybridization takes place at 50-85°C. Each cycle comprises a denaturation treatment and a thermal hybridization treatment which in total is from about one to five minutes long. Typically, the ligation detection reaction involves repeatedly denaturing and hybridizing for 2 to 50 cycles. The total time for the ligase detection reaction process is 1 to 250 minutes.

The oligonucleotide probe sets can be in the form of ribonucleotides, deoxynucleotides, modified ribonucleotides, modified deoxyribonucleotides, modified phosphate-sugar-backbone oligonucleotides, nucleotide analogs, and mixtures thereof.

In one variation, the oligonucleotides of the oligonucleotide probe sets each have a hybridization or melting temperature (i.e. Tₘ) of 66-70°C. These oligonucleotides are 20-28 nucleotides long.

The oligonucleotide probe sets, as noted above, have a reporter label suitable for detection. Useful labels include chromophores, fluorescent moieties, enzymes, antigens, heavy metals, magnetic probes, dyes, phosphorescent groups, radioactive materials, chemiluminescent moieties, and electrochemical detecting moieties.

The product of the ligase detection reaction can be detected in either of two formats. These are fully described in WO 98/03673, to Barany et al.

In one of these formats, ligase detection reaction products are detected by capillary or gel electrophoresis. Alternatively, ligation products can be detected on an array by specific hybridization to a complementary sequence on the array.

The ligation detection reaction mixture may include a carrier DNA, such as salmon sperm DNA.

The hybridization step in the ligase detection reaction, which is preferably a thermal hybridization treatment discriminates between nucleotide sequences based on a distinguishing nucleotide at the ligation junctions. The difference between the target nucleotide sequences can be, for example, a single nucleic acid base difference, a nucleic acid deletion, a nucleic acid insertion, or rearrangement. Such sequence differences involving more than one base can also be detected. Preferably, the oligonucleotide probe sets have substantially the same length so that they hybridize to target nucleotide sequences at substantially similar hybridization conditions. As a result, the process of the present invention is able to detect infectious diseases, genetic diseases, and cancer. It is also useful in environmental monitoring, forensics, and food science.

A wide variety of infectious diseases can be detected by the process of the present invention. Typically, these are caused by bacterial, viral, parasite, and fungal infectious agents. The resistance of various infectious agents to drugs can also be determined using the present invention.

Bacterial infectious agents which can be detected by the present invention include *Escherichia coli, Salmonella, Shigella, Klebsiella, Pseudomonas, Listeria monocytogenes, Mycobacterium tuberculosis, Mycobacterium avium-intracellulare, Yersinia, Francisella, Pasteurella, Brucella, Clostridia, Bordetella pertussis,* Bacteroides, *Staphylococcus aureus, Streptococcus pneumonia,* B-Hemolytic strep., *Corynebacteria, Legionella, Mycoplasma, Ureaplasma, Chlamydia, Neisseria gonorrhea, Neisseria meningitides, Hemophilus influenza, Enterococcus faecalis, Proteus vulgaris, Proteus mirabilis, Helicobacter pylori, Treponema palladium, Borrelia burgdorferi, Borrelia recurrentis, Rickettsial* pathogens, *Nocardia,* and *Acitnomycetes.*

Fungal infectious agents which can be detected by the present invention include *Cryptococcus neoformans, Blastomyces dermatitidis, Histoplasma capsulatum, Coccidioides immitis, Paracoccidioides brasiliensis, Candida albicans, Aspergillus fumigautus, Phycomycetes (Rhizopus), Sporothrix schenckii, Chromomycosis,* and *Maduromycosis.*

Viral infectious agents which can be detected by the present invention include human immunodeficiency virus, human T-cell lymphocytotrophic virus, hepatitis viruses (e.g., Hepatitis B Virus and Hepatitis C Virus), Epstein-Barr Virus, cytomegalovirus, human papillomaviruses, orthomyxo viruses, paramyxo viruses, adenoviruses, corona viruses, rhabdo viruses, polio viruses, toga viruses, bunya viruses, arena viruses, rubella viruses, and reo viruses.

Parasitic agents which can be detected by the present invention include *Plasmodium falciparum, Plasmodium malaria, Plasmodium vivax, Plasmodium ovale, Onchoverva volvulus, Leishmania, Trypanosoma* spp., *Schistosoma* spp., *Entamoeba histolytica, Cryptosporidum, Giardia* spp., *Trichimonas* spp., *Balatidium coli, Wuchereria bancrofti, Toxoplasma* spp., *Enterobius vermicularis, Ascaris lumbricoides, Trichuris trichiura, Dracunculus medinesis,* trematodes, *Diphyllobothrium latum, Taenia* spp., *Pneumocystis carinii,* and *Necator americanis.*

The present invention is also useful for detection of drug resistance by infectious agents. For example, vancomycin-resistant *Enterococcus faecium,* methicillin-resistant *Staphylococcus aureus,* penicillin-resistant *Streptococcus pneumoniae,* multi-drug resistant *Mycobacterium tuberculosis,* and AZT-resistant human immunodeficiency virus can all be identified with the present invention.

Genetic diseases can also be detected by the process of the present invention. This can be carried out by prenatal or post-natal screening for chromosomal and genetic aberrations or for genetic diseases. Examples of detectable genetic diseases include: 21 hydroxylase deficiency, cystic fibrosis, Fragile X Syndrome, Turner Syndrome, Duchenne Muscular Dystrophy, Down Syndrome or other trisomies, heart disease, single gene diseases, HLA typing, phenylketonuria, sickle cell anemia, Tay-Sachs Disease, thalassemia, Klinefelter Syndrome, Huntington Disease, autoimmune diseases, lipidosis, obesity defects, hemophilia, inborn errors of metabolism, and diabetes.

Cancers which can be detected by the process of the present invention generally involve oncogenes, tumor suppressor genes, or genes involved in DNA amplification, replication, recombination, or repair. Examples of these include: BRCA1 gene, p53 gene, APC gene, Her2/Neu amplification, Bcr/Ab1, K-ras gene, and human papillomavirus Types 16 and 18. Various aspects of the present invention can be used to identify amplifications, large deletions as well as point mutations and small deletions/insertions of the above genes in the following common human cancers: leukemia, colon cancer, breast cancer, lung cancer, prostate cancer, brain tumors, central nervous system tumors, bladder tumors, melanomas, liver cancer, osteosarcoma and other bone cancers, testicular and ovarian carcinomas, head and neck tumors, and cervical neoplasms.

In the area of environmental monitoring, the present invention can be used for detection, identification, and monitoring of pathogenic and indigenous microorganisms in natural and engineered ecosystems and microcosms such as in municipal waste water purification systems and water reservoirs or in polluted areas undergoing bioremediation. It is also possible to detect plasmids containing genes that can metabolize xenobiotics, to monitor specific target microorganisms in population dynamic studies, or either to detect, identify, or monitor genetically modified microorganisms in the environment and in industrial plants.

The present invention can also be used in a variety of forensic areas, including for human identification for military personnel and criminal investigation, paternity testing and family relation analysis, HLA compatibility typing, and screening blood, sperm, or transplantation organs for contamination.

In the food and feed industry, the present invention has a wide variety of applications. For example, it can be used for identification and characterization of production organisms such as yeast for production of beer, wine, cheese, yogurt, bread, etc. Another area of use is with regard to quality control and certification of products and processes (e.g., livestock, pasteurization, and meat processing) for contaminants. Other uses include the characterization of plants, bulbs, and seeds for breeding purposes, identification of the presence of plant-specific pathogens, and detection and identification of veterinary infections.

Desirably, the oligonucleotide probes are suitable for ligation together at a ligation junction when hybridized adjacent to one another on a corresponding target nucleotide sequence due to perfect complementarity at the ligation junction. However, when the oligonucleotide probes in the set are hybridized to any other nucleotide sequence present in the sample, there is a mismatch at a base at the ligation junction which interferes with ligation. Most preferably, the mismatch is at the base at the 3' base at the ligation junction. Alternatively, the mismatch can be at the bases adjacent to bases at the ligation junction.

Before carrying out the ligase detection reaction, in accordance with the present invention, target nucleotide sequences in the sample can be preliminarily amplified. This preferably carried out with polymerase chain reaction. The polymerase chain reaction process is fully described in H. Erlich, et. al, "Recent Advances in the Polymerase Chain Reaction," Science 252: 1643-50 (1991); M. Innis, et. al., PCR Protocols: A Guide to Methods and Applications, Academic Press: New York (1990) and R. Saiki, et. al., "Primer-directed Enzymatic Amplification of DNA with a Thermostable DNA Polymerase," Science 239: 487-91 (1988),

The ligase detection reaction process achieves linear amplification of a target sequence. The ligase chain reaction utilizes essentially the same steps and conditions as the ligase detection reaction to achieve exponential amplification. This greater level of amplification is achieved by utilizing 2 sets of complementary oligonucleotides with each set hybridizing to complementary strands of a target nucleic acid sequence.

In the ligase chain reaction process of the present invention, the presence of a target double stranded nucleic acid formed from first and second complementary target nucleotide sequences is detected in a sample. The target double stranded nucleic acid differs from other nucleotide sequences by one or more single base changes, insertions, deletions, or translocations.

This method involves providing a sample potentially containing a target double stranded nucleic acid formed from first and second complementary nucleotide sequence. This nucleic acid differs from other nucleotide sequences in the sample by one or more single base changes, insertions, deletions, or translocations.

The method further includes providing a first oligonucleotide probe set, characterized by (a) a first oligonucleotide probe having a target specific portion and (b) a second oligonucleotide probe having a target-specific portion. The oligonucleotide probes in the first set are complementary to the first target nucleotide sequence which differs from other nucleotide sequences in the sample by one or more single base changes, insertions, deletions, or translocations. The probes are also suitable for ligation together when hybridized adjacent to one another on the first target nucleotide sequence, but have a mismatch which interferes with such ligation when hybridized to any other nucleotide sequence present in the sample. The method of the present invention also requires providing a second oligonucleotide probe set, characterized by (a) a third oligonucleotide probe having a target specific portion and (b) a fourth oligonucleotide probe having a target-specific portion. The oligonucleotide probes in the second set are complementary to the second target nucleotide sequence which differs from other nucleotide sequences in the sample by one or more single base changes, insertions, deletions, or translocations. The probes of the second set are suitable for ligation together when hybridized adjacent to one another on the second target nucleotide sequence, but have a mismatch which interferes with such ligation when hybridized to any other nucleotide sequence present in the sample.

The sample, the first and second oligonucleotide probe sets, and the thermostable ligase are blended together to form a ligase chain reaction mixture. The ligase chain reaction mixture is subjected to one or more ligase chain reaction cycles comprising a denaturation treatment and a hybridization treatment. During the denaturation treatment, any hybridized oligonucleotides are separated from the target nucleotide sequences. In the hybridization treatment, the oligonucleotide probe sets hybridize at adjacent positions in a base specific manner to their respective target nucleotide sequences, if present in the sample. The probes also ligate to one another to form a ligation product sequence containing the target specific portions connected together with the ligation product sequences for each set being distinguishable from other nucleic acids in the ligase chain reaction mixture. The oligonucleotide probe sets may hybridize to nucleotide sequences in the sample other than their respective target nucleotide sequences but do not ligate together due to a presence of one or more mismatches and individually separate during the denaturation treatment. The presence of ligation product sequences produced as a result of the target nucleotide sequence being present in the sample are then detected.

### EXAMPLES

### Example 1 - Reagents, Media, and Strains

A.11 routine chemical reagents were purchased from Sigma Chemicals (St. Louis, MO) or Fisher Scientific (Fair Lawn, NJ). Restriction enzymes and T4 DNA ligase were obtained from New England Biolabs (Beverly, MA). Oligonucleotide synthesis reagents, DNA sequencing kits, and PCR kits were obtained from Applied Biosystems Division of Perkin-Elmer Corporation (Foster City, CA). dNTPs, BSA (i.e. bovine serum albumin), ATP were purchased from Boehringer-Mannheim (Indianapolis, IN). *Pfu* DNA polymerase was purchased from Stratagene (La Jolla, CA). *E. coli* strain NovaBlue(DE3)pLysS, and plasmid pET11c were purchased from Novagen, Inc. (Madison, WI). Protein assay kit was from Bio-Rad (Hercules, CA). HiTrap Blue affinity column was from Pharmacia (Piscataway, NJ). LB medium was prepared according to standard formula (Sambrook, et al., (1989) Molecular Cloning-A Laboratory Manual, 2nd Ed., Cold Spring Harbor Laboratory Press, Cold Spring Harbor, New York (1994), which is hereby incorporated by reference). Sonication buffer consisted of 50 mM Tris-HCl, pH 8.0 and 1 mM EDTA. TE buffer consisted of 10 mM Tris-HCl, pH 8.0 and 1 mM EDTA. *Tth DNA* ligase and its mutant K294R were purified as previously described (Luo, et al., Nucleic Acids Res, 24(15):3071-3078 (1996), which is hereby incorporated by reference).

### Example 2 - Oligonucleotide Synthesis

Oligonucleotides were synthesized by using a 394 automated DNA synthesizer from Applied Biosystems Division of Perkin-Elmer Corp. PCR and sequencing primers were purified by ethanol precipitation according to instruction manual. The degenerate sense primer 5'-ATC(T/A)(C/G)CGACGC(C/G)-GA(G/A)TA(T/C)GA-3' (SEQ. ID. No. 3) corresponding to amino acids 32-38 (ISDAEYD) (SEQ. ID. No. 4) in the *T. thermophilus* HB8 DNA ligase gene, and antisense primers 5'-CC(C/G)GT(C/G)C(G/T)-(G/C)CC(G/C)AC(C/T)TG(A/G)AA-3' (SEQ. ID. No. 5) and 5'-GCCTTCTC(C/G/A)A(A/G)(T/C)TTG-(C/G)(A/T)(G/C)CC-3' (SEQ. ID. No. 6) corresponding to amino acids 333-339 (FQVGRTG) (SEQ. ID. No. 7) and 641-647 (GSKLEKA) (SEQ. ID. No. 8) were used to amplify DNA ligase gene fragments from *Thermus* strains. Additional PCR and sequencing primers were synthesized as required. PCR amplification primers for cloning *Tsp.* AK16D DNA ligase gene into pET11c vector were 5'-GCGATTTCATATGACCCTAGAGGAGGCCCG-3'(SEQ.ID. No. 9) and 5'-GCGGGATCCGAGGC CTTGGAGAAGCTCTT-3', (SEQ. ID. No. 10) where the *Nde*I and *Bam*HI sites are underlined and the initiation codon in the forward primer is shown in bold. Oligonucleotide substrates for ligation assay were purified on a denaturing sequencing gel (7 M urea/l 0% polyacrylamide) (Applied Biosystems Inc., The complete guide to evaluating and isolating synthetic oligonucleotides, Applied Biosystems Inc., Foster City, CA (1992)). 5'-phosphorylation of oligonucleotides was achieved during synthesis by using Chemical Phosphorylation Reagent (Glen Research, Sterling, VA). Fluorescent group was attached to a 3'-terminus using Fluorescein CPG column (Glen Research).

### Example 3- DNA Amplification, Cloning And Sequence Analysis

Genomic DNAs from *Thermus* strains were isolated as previously described (Cao, et al., Gene, 197:205-214 (1997), which is hereby incorporated by reference). PCR amplifications with degenerate and unique primers and inverse PCR on circularized templates were carried out in a GeneAmp PCR System 9700 thermocycler (Applied Biosystems Division of Perkin Elmer) as described (Wetmur, et al., J Biol Chem, 269(41):25928-25935 (1994), which is hereby incorporated by reference). The nucleotide sequences of amplified ligase fragments were directly determined on an ABI 373 sequencer using ABI PRISM^{™} Dye Terminator Cycle Sequencing Ready Reaction Kit (Perkin-Elmer). Full length *Tsp.* AK16D DNA ligase gene was amplified using PCR amplification primers as described above, digested with *Nde*I and *Bam*HI*,* ligated into the cloning vector pET11c treated with the same pair of restriction enzymes, and transformed into *E*. *coli* strain NovaBlue(DE3)pLysS. Inserts in pET expression vectors were sequenced in both orientations to ensure that the plasmid constructs were free of PCR or ligation error. Nucleic acid and protein sequence analyses were carried out by Clustal method (Higgins, et al., Comput Appl Biosci, 5(2):151-153 (1989), which is hereby incorporated by reference) using MegAlign program of D-VASTAR (Madison, WI).

### Example 4 - Expression and Purification of Tsp. AK16D DNA Ligase

*E. coli* NavaBlue(DE3)pLysS cells containing plasmid pTAK encoding the *Tsp.* AK16D DNA ligase gene from a pET11c construct was propagated overnight at 37°C in LB medium containing 50 µg/ml ampicillin. 25 µg/ml chloramphenicol, and 0.2% glucose. Overnight cultures were diluted 100-fold into the same medium, grown until the optical density of the culture reached 0.5 at 600 nm, then induced by the addition of IPTG to a final concentration of 1mM, and grown for an additional 4 hrs under the same conditions. Cells were collected by centrifugation, frozen/thawed at-20°C/23°C, disrupted by sonication, and clarified by centrifugation as previously described (Wetmur, et al., J Biol Chem, 269(41):25928-25935 (1994), which is hereby incorporated by reference). The resulting supernatants were heated at 70°C for 15 min to denature thermolabile *E. coli* proteins, placed on ice for 30 min to aggregate the denatured proteins, and cleared of denatured proteins by microcentrifugation for 15 min at 4°C. The partially pure DNA ligase was further purified by chromatography using 1 ml HiTrap Blue affinity column. Briefly, the column containing *Tsp.* AK16D DNA ligase was washed extensively with TE buffer (pH 7.8) containing 0.1 M NaOAc, and the ligase was eluted with TE buffer (pH 7.8) containing 2 M NaCl. After dialysis against TE buffer (pH 8.0) containing 0.2 M KCl and concentration using Centricon-30 (Amicon), protein concentration was assayed by the Bradford method with reagents supplied by Bio-Rad protein assay kit. The amount of protein was determined using BSA as the standard. The purity of the ligase was verified through 7.5% SDS (i.e. sodium dodecyl sulfate)-PAGE (i.e. polyarcylamide gel electrophoresis) analysis followed by visualizing the overloaded gel with routine Coomassie Brilliant Blue R staining.

### Example 5 - Substrates And Ligation Assay

The oligonucleotide perfect match substrate was formed by annealing two short oligonucleotides (33-mer for LP3'C (SEQ. ID. No. 11) and 30-mer for Com3F (SEQ. ID. No. 12)) with a 59-mer complementary oligonucleotide (Glg). Oligonucleotides LP3'C and Glg (SEQ. ID. No. 14) were in 1.5-fold excess so that the all the 3' Fam labeled Com3F represented nicked substrates (sec Luo, et al., Nucleic Acids Res, 24(15):3071-3078 (1996), which is hereby incorporated by reference). The T/G mismatch substrate was formed by annealing LP3'T (SEQ. ID. No. 13), which introduced a single base-pair mismatch at the 3'-end of the nick junction, along with Com 3'F to the complementary strand (Glg). The nicked DNA duplex substrates were formed by denaturing DNA probes at 94°C for 2 min followed by re-annealing at 65°C for 2 min in ligation buffer. The sequences of the oligonucleotides were listed below (p represents 5' phosphate group):

Ligation mixtures (20 µl) containing indicated amount of DNA ligase and match or mismatch substrate in the ligase buffer (20 mM Tris-HCl, pH 7.6 at room temperature; 10 mM MgCl₂; 100 mM KCl; 10 mM DTT (i.e. dithiothreitol); 1 mM NAD⁺; and 20 mg/ml BSA) were incubated at 65°C for a predetermined time. Reactions were terminated by the addition of an equal volume of stop solution (i.e. 50 mM EDTA, 80% formamide, and 1% Blue Dextran). Samples (5 µl) were electrophoresed through an 8 M urea-10% polyacrylamide GeneScan gel according to instructional manual (Perkin Elmer). The unreacted substrates were represented by the 30-mer com3F and products were represented by a ligated 63-mer in the case of the match substrate. Both the remaining substrates and ligated products were quantified using GeneScan analysis software 672 (version 2.0. Perkin Elmer).

### Example 6 - Steady State Kinetics

Steady state kinetic constants were determined by measuring initial rates of the ligation reaction at a given substrate concentration (nicked DNA duplex substrate concentration ranging from 25-400 nM) and a given ligase concentration (12.5 pM for both *Tth* and *Tsp.* AK16D) in 100 µl reaction volume at 65°C. A 5 µl aliquot was removed at 0, 2, 4, 6, 8, 10 min, and mixed with 5µl of stop solution. The remaining substrate was separated from ligated product by GeneScan gel as described above. Initial rates of the ligation reactions were calculated from the generation of ligated product over time. The *K*ₘ and *k*_{cat} values were determined using computer software Ultrafit (Biosoft, Ferguson, MO).

### Example 7 - Sequence Analysis Of Seven Thermus Ligase Genes

Amino acid sequence alignment of five Gram negative bacterial NAD⁺-dependent DNA ligases indicates that *Tth* ligase is 93% identical to *Thermus scotoductus* ligase, 49% to *Rhodothermus marinus* ligase, 48% to *E. coli* ligase, and 38% to *Zymomonas mobilis* based on sequence data retrieved from GeneBank. Degenerate primers corresponding to highly conserved regions of these ligases were used to amplify fragments of ligase genes from seven *Thermus* strains which represent a worldwide collection: *Thermus flavus* from Japan (SEQ. ID. No. 16), *Thermus aquaticus* YT-1 (SEQ. ID. No. 15) and *Thermus sp.* AK16D from Yellowstone National Park in the United States, *Thermus filiformis* Tok4A2 (SEQ. ID. No. 17) and *Thermus filiformis* Tok6A1 (SEQ. ID. No. 18) from New Zealand, *Thermus sp.* SM32 (SEQ. ID. No. 19) from Azores, and *Thermus sp.* Vil3 (SEQ. ID. No. 20) from Portugal. The sequences of amplified ligase fragments ranging from 1.4 to 1.6 kb were determined by directly sequencing the PCR products using an ABI 373 automated sequencer. *Thermus* ligases, in general, were highly conserved during evolution as demonstrated by 85%-98% sequence identity. In contrast, the amino acid sequences of the restriction endonuclease *Taq*I and its isoschizomers from the identical strains show only 50-70% aa identities (Cao, et al., Gene, 197:205-214 (1997), which is hereby incorporated by reference)*. Thermus* ligases in general show 30-40% sequence identities as compared with DNA ligases from other bacteria. The sequence divergence is slightly higher among the different geographic groups than within the same group, which may reflect random drift or adaptation to their respective local environments (Figure 1). *Thermus flavus, Thermus filiformis* Tok4A2, *Thermus filiformis* Tok6A1, *Thermus sp.* SM32, *Thermus sp.* Vil3, *Thermus aquaticus* YT-1, and *Thermus sp.* AK16D (SEQ. ID. No. 14) ligases shared 98.2%, 89.9%, 89.5%, 89.8%, 88.3%, 88.2%, 88.1% with *Thermus thermophilus* HB8 DNA ligase, respectively. The adenylation site of the enzymes (¹¹⁸KXDG where X is in general a hydrophobic residue), as identified by site-directed mutagenesis of *Tth* DNA ligase, is completely identical among all *Thermus* ligases, furthermore, the flanking sequences of the adenylation motif are also identical except *Tsp.* AK16D in which the aa residues ¹¹⁷H before the ¹¹⁸K is substituted by an ¹¹⁸R (Figure 1B). In non*-Thermus* NAD⁺-dependent ligases discovered to date, the corresponding position is either a Pro or a Leu. The two isolates from Japan can be distinguished from the other *Thermus* strains by a 3-aa-insertion at position 234.

### Example 8 - Cloning, Expression And Purification Of DNA Ligase From Tsp. AK16D

To maximize the chance of finding a *Thermus* ligase with novel properties, *Tsp.* AK16D ligase was chosen which showed the least sequence identity as compared with *T. thermophilus* ligase. To obtain the complete sequence of the ORF (i.e. open reading frame), the fragments of the N- and C-terminus of the gene were amplified by inverse PCR and were subject to direct sequencing. The complete ORF of the *Thermus sp.* AK16D ligase gene consists of 674 amino acids, as compared to 676 aa for *Tth* ligase and 674 aa for T. scot ligase (Figure 1C). The full-length *Thermus sp.* AK16D ligase gene was PCR amplified using *Pfu* polymerase and cloned into expression plasmid pET11c (Novagen). The integrity of the insert containing the ligase gene was verified by DNA sequencing. The pET11c plasmid expressing *Tsp.* AK16D ligase was transformed into competent *E*. *coli* cells NovaBlue(DE3)pLysS. Production of ligases was induced by adding IPTG to 1 mM final concentration. *Tsp.* AK16D ligase protein was expressed to approximately 10% of total cellular proteins (Figure 2, lane 3). Heating at 70°C for 15 minutes denatured most of *E. coli* proteins while leaving the thermostable ligases as the dominant band (Figure 2, lane 4). A cibacron blue based affinity chromatography (Pharmacia) further removed residual *E. coli* proteins and nucleic acids, yielding apparently homogenous *Tsp.* AK16D ligase protein as judged by Coomassie staining (Figure 2, lane 5).

### Example 9 - Salt, pH, and NAD⁺ Dependence Of The Ligation Reaction

Figure 3A depicts the pH dependence of ligase activity of *Tth* and *Tsp.* AK16D ligase proteins. The shape of the pH dependence curves of *Tth* ligase and *Tsp.* AK16D ligase is essentially superimposable. The optimal pH is 8.5 for both *Tth* ligase and *Tsp.* AK16D ligase with greater than 80% activity observed between pH 7.8 and 9.5. The identity of pH effect suggests that both of the ligases possess similar local environment at their catalytic center, which is in agreement with the degree of sequence conservation between the two ligases. Figure 3B depicts the salt concentration dependence of ligase activity of *Tth* and *Tsp.* AK16D ligase proteins. The optimum KCI concentration for *Tth* ligase and *Tsp.* AK16D ligase are 100 and 50 mM, respectively. Figure 3C depicts the NAD⁺ concentration dependence of ligase activity of *Tth* and *Tsp.* AK16D proteins. The optimum NAD⁺ concentration is 1 mM for both *Tth* ligase and *Tsp.* AK16D ligase. The similarity of the NAD profiles is in keeping with the highly conserved nature of the N-terminal domain of the ligases which is involved in NAD⁺ binding.

### Example 10 - Effects Of Divalent Metals On The Ligation Reaction

Divalent metal ion is indispensable for each of the three steps in a ligation reaction: (i) adenylation of a lysine residue in the adenylation motif KXDG; (ii) transfer of the adenylate to the 5' phosphate to form a DNA-adenylate intermediate; and (iii) formation of the phosphodiester bond with the release of adenosine monophosphate (AMP). In general, Mg²⁺ is the preferred metal ion for both ATP-dependent and NAD⁺-dependent ligases. Mg²⁺ was substituted with alkaline earth metal ion Ca²⁺ and commonly studied period 4 transition metal ions. *Tth* and *Tsp.* AK16D ligases could use Mn²⁺ as an alternative metal cofactor to support ligation activity (Figure 4). Both enzymes were less active with Ca²⁺, while Co²⁺, Ni²⁺, Cu²⁺, and Zn²⁺ failed to support ligation. In comparison, ATP-dependent ligase from Hin (i.e. *Haemophilus influenzae)* uses only Mg²⁺ and Mn²⁺ as the metal cofactor for nick closure but not Ca^{2t}, Co²⁺, Cu²⁺, and Zn²⁺ (Cheng. et al., Nucleic Acids Res, 25(7):1369-1374 (1997) ; ATP-dependent ligase from *Chlorella* virus PBCV-1 can use Mg²⁺, Mn²⁺, and Co²⁺ but not Ca²⁺, Cu²⁺, and Zn²⁺ (Ho, et al., J Virol, 71(3):1931-1937 (1997), which is hereby incorporated by reference). Using Ca²⁺ as the metal cofactor. *Thermus* enzymes were able to convert most of the substrate into the DNA-adenylate intermediate. However, the rates of nick closure were reduced which led to the accumulation of the DNA-adenylate intermediate (Figure 4B). A small amount of the intermediate was observed with Ni²⁺; however, ligation product was not observed at the current detection level, suggesting that Ni²⁺ could not support the nick closure step (Figure 4B). To further compare the relative activity of the two *Thermus* ligases with Mg²⁺ and Mn²⁺, the generation of ligation product was first monitored over a 20-min time period. As shown in Figure 5, the *Thermus* enzymes were consistently more active with Mg²⁺ than with Mn²⁺. Second, ligation activity up to 40 mM Mg²⁺ or Mn²⁺ concentrations (Figure 6) was assayed. Both of the enzymes responded sensitively to the change of the metal ion concentration in the reaction mixture. At high M²⁺ concentrations, the high ionic strength may inhibit the enzyme activity, consistent with KCl dependence profile (Figure 4). Similar to the time-course results, the *Thermus* enzymes were more active with Mg²⁺ than with Mn²⁺ (Figure 6). The discrepancy on the relative activity of *Thermus* ligases between this study and an earlier report may be due to use here of cloned enzymes while the earlier work used purified native enzyme (Takahashi, et al., J Biol Chem, 259(16):10041-10047 (1984), which is hereby incorporated by reference).

### Example 11 - Steady State Kinetics

The steady state kinetic constants were measured by monitoring the formation of fluorescently labeled ligation product over time using substrate concentrations spanning estimated *K*m values (Table 1).

**Table 1. Steady state kinetics of Tth and Tsp. AK16D ligase^{a}**

| Ligase | *K*ₘ (nM) | *k*_{cat} (min⁻¹) | *k*_{cat}/*K*ₘ (M⁻¹ s⁻¹) |
|---|---|---|---|
| Tth | 87 | 56 | 1.1 x 10⁷ |
| *Tsp.AK*16D | 104 | 38 | 0.63 x 10⁷ |

| | | | |
|---|---|---|---|
| ^{a}: Results represent the average of at least three experiments. | | | |

The steady state properties of *Tsp.* AK16D ligase were similar to *Tth* ligase, indicating that the catalytic channels are highly conserved in *Thermus* ligases. The average *K*m value of about 90 nM for *Thermus* ligases is similar to the *K*m value of 50 nM for *E. coli* ligase (Modrich, et al., J Biol Chem. 248(21):7495-7501 (1973), which is hereby incorporated by reference) and about 10-fold higher than vaccinia virus ATP-dependent ligase (Sekiguchi, et al., Nucleic Acids Res. 25(4):727-734 (1997) ). The average *k*cat value of about 45 turnovers per min for *Thermus* ligases is higher than the kcat value of 28 turnovers per min for *E. coli* ligase (Modrich, et al., J. Biol Chem, 248(21):7495-7501 (1973), which is hereby incorporated by reference).

### Example 12- Ligation Of Gapped Or Inserted DNA Duplex Substrates

Gapped substrates were formed by deleting one or two nt from the 3' hydroxyl site of oligonucleotide LP3'C, and inserted substrates were formed by adding one or two nt at the 3' hydroxyl site of oligonucleotide LP3'C. Gapped or inserted duplexed DNA sequences are distinctively different from normal nicked substrate. Under our experimental conditions, no ligation was detectable with 1-nt (i.e. nucleotide) or 2-nt gapped or 2-nt insertion substrates for either *Tth* or *Tsp.* AK16D ligase (Figure 7A). As for 1-nt insertion substrates, only A insertion gave a trace amount of ligated products for both ligases (Figure 7A). All other 1-nt insertions at the ligation junction could not be ligated. In contrast, Hin ligase and *Chlorella* ligase demonstrate observable ligation with 1-nt gap (Ho, et al., J Virol, 71(3):1931-1937 (1997) and Cheng, et al., Nucleic Acids Res, 25(7):1369-1374 (1997), which is hereby incorporated by reference). In the case of vaccinia ligase, the ligation of 1-nt gap is negligible but the formation of DNA-adenylate intermediate is significant, suggesting the major impact of using 1-nt gapped substrate is on nick closure (Shuman, S., Biochemistry, 34(49):16138-16147 (1995), which is hereby incorporated by reference). The formation of DNA-adenylate intermediate with the *Thermus* enzymes was not observed, suggesting that most of the gapped or inserted substrates may have abolished the possibility of completing the second step in the ligation cycle - adenylation of DNA substrate at the 5' phosphate. The 1-nt A insertion mis-ligation could be due to slippage (Figure 7B). Although *Thermus* ligase slippage is far less than *Thermus* DNA polymerase, it does occur at a low frequency. Given the fact that the adjacent nt is a T, the slippage could have occurred at 5' phosphate side where a 5'A/C mismatch is ligated (Luo, et al., Nucleic Acids Res, 24(15):3071-3078 (1996), which is hereby incorporated by reference). It is unlikely that the enzyme tolerates slippage on the 3' side , because a 1 nt C insertion did not yield detectable ligation product (Figure 7).

### Example 13 - Thermus DNA Ligase Fidelity

*Tth* DNA ligase is more discriminative when the mismatch is located at the 3' side of the nick junction. 3'G/T or 3'T/G is the only mismatch that shows observable mismatch ligation (Luo, et al., Nucleic Acids Res, 24(15):3071-3078 (1996), which is hereby incorporated by reference). To evaluate the fidelity of the cloned *Tsp.* AK16D ligase, the rate ratio of match over 3'T/G mismatch ligation was compared with wild-type and K294R mutant *Tth* DNA ligases along with T4 ligase from a commercial source (Table 2).

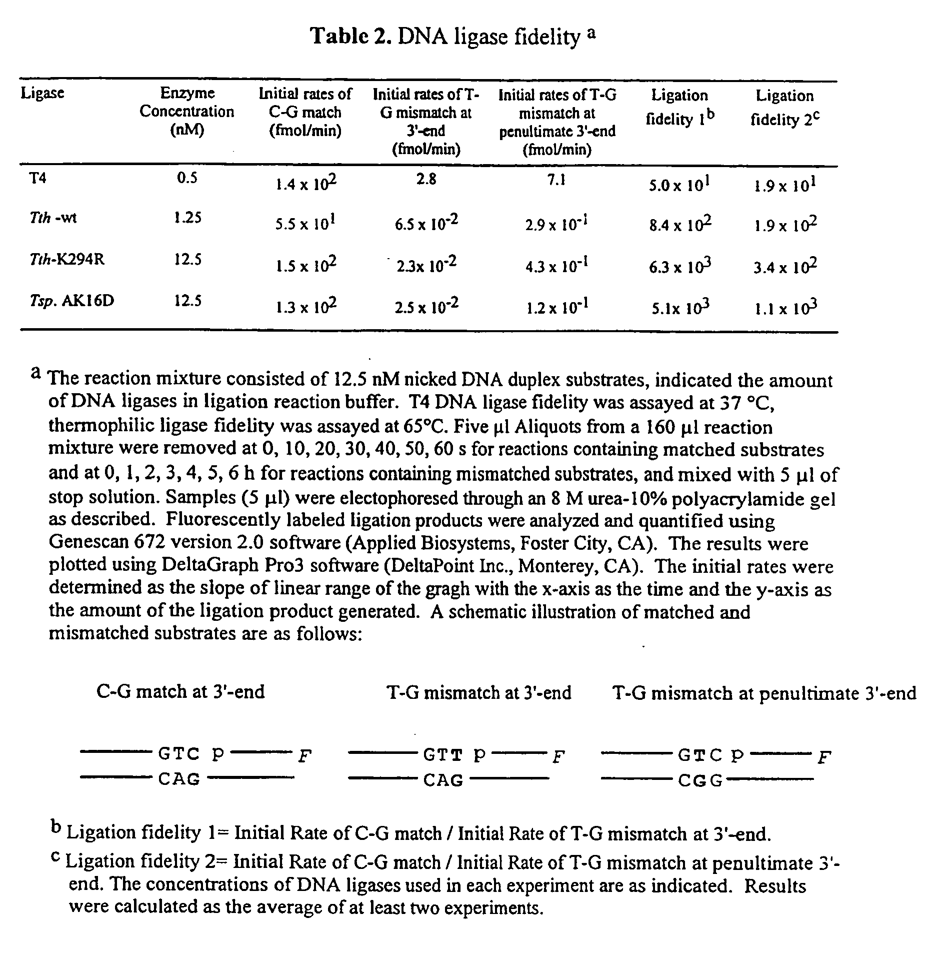

T4 ligase demonstrated high catalytic efficiency toward both match and 3'T/G mismatch substrate such that a ligation fidelity of 50 was obtained. *Thermus* ligases appeared to be less efficient in match ligation as evidenced by the requirement of higher enzyme concentration to achieve comparable match ligation rate. However, under the same assay conditions, *Thermus* enzymes were far less prone to ligate a 3'T/G mismatch. As a result, the fidelity of *Thermus* enzymes was 17- to 126-fold higher than T4 ligase (Table 2, Ligation fidelity 1). The fidelity of the newly cloned *Tsp.* AK16D ligase was similar to K294R *Tth* mutant but 6-fold higher than wild-type *Tth* enzyme. A DNA-adenylate intermediate was observed with 3'T/G mismatch ligation, suggesting that a mismatch at the 3' ligation junction imposes substantial constraints on the ability of *Thermus* ligases to close the nick, thereby limiting the turnover of DNA-adenylate intermediate into ligated product and free AMP (the third step of ligation cycle). The effects of moving the T/G mismatch one base-pair away from the ligation junction was further examined. The rates of ligation with a T/G mismatch at the penultimate 3' end in general improved several-fold as compared with the T/G mismatch at the 3' end of the ligation junction. However, the ligation rates were still much slower than those of match ligation, emphasizing the importance of nucleotide complementarity near the ligation junction as well as the ultimate critical role of the perfect base-pair at the 3' end in controlling ligation reaction. Consequently, the ligation fidelity when the mismatch was at the second position from the 3' side (ligation fidelity 2) was lower than that when the mismatch was located immediately at the ligation junction. It is noteworthy that the *Tsp.* AKI6D enzyme maintains extremely high fidelity (1.1 x 10³) even when the mismatch is at the penultimate position, further underscoring the discriminative power of this new *Thermus* ligase.

### Example 14 - Thermostable DNA Ligase Fidelity In The Presence Of Mn²⁺

Many enzymes such as DNA polymerase and restriction endonucleases demonstrate relaxed specificity when Mn²⁺ is used as the metal cofactor. The influence of metal ion substitution on ligase fidelity has not been fully investigated although it is known that Mn²⁺ can be used as an alternative metal cofactor for a ligation reaction ((Ho, et al., J Virol, 71 (3):1931-1937 (1997) and Cheng, et al., Nucleic Acids Res, 25(7):1369-1374 (1997), which is hereby incorporated by reference). The reaction rates of the match and mismatch ligation for *Tsp.* AK16D ligase and *Tth* ligase were determined. As shown in Table 3, the match ligation rates were higher with Mg²⁺ than with Mn²⁺ (Table 3), in agreement with the consistent high ligation rate under various Mg²⁺ conditions (Figure 4-6).

**Table 3. DNA ligase fidelity with Mn^{2+a}**

| Ligase | Concentration (nM) | Initial rate of C-G match (fmol/min) | Initial rate of T-G mismatch (fmol/min) | Ligation fidelity |
|---|---|---|---|---|
| *Tth*-wt | 1.25 | 2.6 X 10¹ | 3.7 X 10⁻¹ | 7.0 X 10¹ |
| *Tsp.* AK16D | 12.5 | 9.5 X 10¹ | 1.1 X 10⁻¹ | 8.6 X 10² |

| | | | | |
|---|---|---|---|---|
| ^{a} Reaction conditions were identical to those in Table 2, except that 10 mM Mn²⁺ was used in place of Mg²⁺. Ligation fidelity was defined as the ratio of Initial Rate of C-G match divided by Initial Rate of T-G mismatch at 3'-end. Results were calculated as the average of at least two experiments. | | | | |

The mismatch ligation rate of *Tth* ligase was about six-fold higher with Mn²⁺ than with Mg²⁺ while that of *Tsp.* AK1 6D ligase was about 4-fold higher. Thus, as with other previously studied DNA enzymes, DNA ligases also demonstrate relaxed specificity when Mg²⁺ is substituted with Mn²⁺. As a result, the fidelity factors of *Tth* ligase and *Tsp.* AK16D ligase were reduced 12- and 6-fold, respectively (Tables 2-3). Remarkably, the *Tsp.* AK16D enzyme retains 12-fold higher fidelity against mismatch ligation than the *Tth* enzyme. In contrast to using Mg²⁺ as the metal cofactor, *Tth* ligase did not generate DNA-adenylate intermediate during 3T/G mismatch ligation with Mn²⁺. This observation suggests that the nick closure of a 3'T/G mismatch by the *Tth* enzyme is accelerated with Mn²⁺. On the other hand, the *Tsp.* AK16D enzyme accumulated DNA-adenylate intermediate during 3'T/G mismatch ligation with either Mg²⁺ or Mn²⁺. These results indicate that the nick closure of a 3'T/G mismatch with Mn²⁺ by *Tsp.* AK16D DNA ligase remains as the rate-limiting step, which accounts for the higher fidelity of this enzyme.

Studies on *Tth* DNA ligase has deepened understanding of thermostable ligases and has reaffirmed the common theme of ligation - adenylation of ligase at the KXDG motif (Luo, et al., Nucleic Acids Res, 24(15):3079-3085 (1996), which is hereby incorporated by reference). This study reveals that *Thermus* ligases may differ from each other as to substrate specificity despite their highly identical primary protein sequences. A highly homologous structure can be anticipated from various *Thermus* ligases, but subtle local environments may dictate the probability of accepting a particular mismatch as the substrate. The fidelity of the *Thermus* ligases may be determined by multiple domains, multiple motifs and/or multiple sequence elements. In comparison of *Tth* and *Tsp.* AK16D ligases, one can find that although K294R (in an identical local environment, see Figure 1B) enhances the fidelity of *Tth* ligase (Luo, et al., Nucleic Acids Res, 24(15):3071-3078 (1996), which is hereby incorporated by reference), *Tsp.* AK16D ligase with a K in this position can still demonstrate superior mismatch discrimination. Additional sequence elements remain to be uncovered. The R substitution at the adjacent position to the KXDG motif may have an effect on the *Tsp.* AK16D ligase's specificity, because studies on *Chlorella* ligase has emphasized the importance of occupying AMP binding pocket for nick recognition (Sriskanda, et al., Nucleic Acids Res, 26(2):525-531 (1998)). The accumulation of DNA-adenylate intermediate with some divalent metal ions by *Tsp.* AK16D ligase asserts that the nick closure step of a ligation reaction can be sensitive to the selection of metal ions, gapped substrates and mismatch substrates. More structural and functional studies on *Tsp.* AK16D ligase could reveal how this enzyme achieves high fidelity with different substrates and different metal ions.

Although the invention has been described in detail for the purpose of illustration, it is understood that such details are solely for that purpose and that variations can be made therein by those skilled in the art without departing from the scope of the invention which is defined by the following claims.

## Claims

1. An isolated thermostable ligase, wherein the thermostable ligase has the amino acid sequence of SEQ. ID. No. 1.

2. An isolated DNA molecule encoding a thermostable ligase having the amino acid sequence of SEQ. ID. No. 1.

3. The isolated DNA molecule according to claim 2, wherein said DNA molecule has the nucleotide sequence of SEQ. ID. No. 2.

4. A DNA expression vector transduced with the heterologous DNA molecule according to claim 2.

5. A DNA expression vector transduced with the heterologous DNA molecule according to claim 3.

6. A host cell transduced with the heterologous DNA molecule according to claim 2.

7. A host cell transduced with the heterologous DNA molecule according to claim 3.

8. A method for detecting, in a sample, a target nucleotide sequence which differs from other nucleotide sequences in the sample by one or more single base changes, insertions, deletions, or translocations, said method comprising:
providing a sample potentially containing a target nucleotide sequence which differs from other nucleotide sequences in the sample by one or more single base changes, insertions, deletions, or translocations;
providing one or more oligonucleotide probe sets, each **characterized by** (a) a first oligonucleotide probe having a target specific portion and (b) a second oligonucleotide probe having a target-specific portion, wherein the oligonucleotide probes in a particular set are suitable for hybridization to a target nucleotide sequence which differs from other nucleotide sequences in the sample by one or more single base changes, insertions, deletions, or translocations and for ligation together when hybridized adjacent to one another on the target nucleotide sequence, but have a mismatch which interferes with such ligation when hybridized to any other nucleotide sequence present in the sample;
providing a thermostable ligase according to claim 1;
blending the sample, the one or more oligonucleotide probe sets, and the thermostable ligase to form a ligase detection reaction mixture;
subjecting the ligase detection reaction mixture to one or more ligase detection reaction cycles comprising a denaturation treatment, wherein any hybridized oligonucleotides are separated from the target nucleotide sequence, and a hybridization treatment, wherein the oligonucleotide probe sets hybridize at adjacent positions in a base specific manner to their respective target nucleotide sequences, if present in the sample, and ligate to one another to form a ligation product sequence containing the target specific portions connected together with the ligation product sequences for each set being distinguishable from other nucleic acids in the ligase detection reaction mixture, wherein the oligonucleotide probe sets may hybridize to a nucleotide sequence in the sample other than their respective target nucleotide sequences but do not ligate together due to a presence of one or more mismatches and individually separate during the denaturation treatment; and
detecting the presence of ligation product sequences produced as a result of the target nucleotide sequence being present in the sample.

9. A method according to claim 8 further comprising:
amplifying, prior to said blending, the target nucleotide sequence present in the sample.

10. A method according to claim 9, wherein said amplifying is carried out by polymerase chain reaction.

11. A method for detecting, in a sample, a target double stranded nucleic acid formed from first and second complementary target nucleotide sequences which differ from other nucleotide sequences by one or more single base changes, insertions, deletions, or translocations, said method comprising:
providing a sample potentially containing a target double stranded nucleic acid formed from first and second complementary nucleotide sequences which differs from other nucleotide sequences in the sample by one or more single base changes, insertions, deletions, or translocations;
providing a first oligonucleotide probe set, **characterized by** (a) a first oligonucleotide probe having a target specific portion and (b) a second oligonucleotide probe having a target-specific portion, wherein the oligonucleotide probes in the first set are complementary to the first target nucleotide sequence which differs from other nucleotide sequences in the sample by one or more single base changes, insertions, deletions, or translocations and are suitable for ligation together when hybridized adjacent to one another on the first target nucleotide sequence, but have a mismatch which interferes with such ligation when hybridized to any other nucleotide sequence present in the sample;
providing a second oligonucleotide probe set, **characterized by** (a) a third oligonucleotide probe having a target specific portion and (b) a fourth oligonucleotide probe having a target-specific portion, wherein the oligonucleotide probes in the second set are complementary to the second target nucleotide sequence which differs from other nucleotide sequences in the sample by one or more single base changes, insertions, deletions, or translocations and are suitable for ligation together when hybridized adjacent to one another on the second target nucleotide sequence, but have a mismatch which interferes with such ligation when hybridized to any other nucleotide sequence present in the sample;
providing a thermostable ligase according to claim 1;
blending the sample, the first and second oligonucleotide probe sets, and the thermostable ligase to form a ligase chain reaction mixture;
subjecting the ligase chain reaction mixture to one or more ligase chain reaction cycles comprising a denaturation treatment, wherein any hybridized oligonucleotides are separated from the target nucleotide sequences, and a hybridization treatment, wherein the oligonucleotide probe sets hybridize at adjacent positions in a base specific manner to their respective target nucleotide sequences, if present in the sample, and ligate to one another to form a ligation product sequence containing the target specific portions connected together with the ligation product sequences for each set being distinguishable from other nucleic acids in the ligase chain reaction mixture, wherein the oligonucleotide probe sets may hybridize to a nucleotide sequences in the sample other than their respective target nucleotide sequences but do not ligate together due to a presence of one or more mismatches and individually separate during the denaturation treatment; and
detecting the presence of ligation product sequences produced as a result of the target nucleotide sequence being present in the sample.

## Patentansprüche

1. Isolierte thermostabile Ligase, **dadurch gekennzeichnet, dass** die thermostabile Ligase die Aminosäuresequenz mit der Sequenzidentifizierungsnummer 1 aufweist.

2. Isoliertes DNA-Molekül, das eine thermostabile Ligase kodiert, die die Aminosäuresequenz mit der Sequenzidentifizierungsnummer 1 aufweist.

3. Isoliertes DNA-Molekül nach Anspruch 2, **dadurch gekennzeichnet, dass** das DNA-Molekül die Nukleotidsequenz mit der Sequenzidentifizierungsnummer 2 aufweist.

4. DNA-Expressionsvektor, der durch Transduktion mit dem heterologen DNA-Molekül nach Anspruch 2 übertragen wird.

5. DNA-Expressionsvektor, der durch Transduktion mit dem heterologen DNA-Molekül nach Anspruch 3 übertragen wird.

6. Wirtszelle, die durch Transduktion mit dem heterologen DNA-Molekül nach Anspruch 2 übertragen wird.

7. Wirtszelle, die durch Transduktion mit dem heterologen DNA-Molekül nach Anspruch 3 übertragen wird.

8. Verfahren zum Nachweis einer Nukleotid-Zielsequenz in einem Prüfgut, die sich von anderen Nukleotidsequenzen in dem Prüfgut durch eine oder mehrere einzelne Basenveränderungen, Baseneinfügungen, Basendeletionen oder -translokationen unterscheidet, wobei das Verfahren folgende Verfahrensschritte umfasst:
Bereitstellen von Prüfgut, das potentiell eine Nukleotid-Zielsequenz enthält, die sich von anderen Nukleotidsequenzen in dem Prüfgut durch eine oder mehrere einzelne Basenveränderungen, Baseneinfügungen, Basendeletionen oder -translokationen unterscheidet;
Bereitstellen eines oder mehrerer Oligonukleotid-Sondensätze, von denen jeder Satz durch (a) eine erste Oligonukleotid-Sonde mit einem zielspezifischen Abschnitt und (b) eine zweite Oligonukleotid-Sonde mit einem zielspezifischen Abschnitt **gekennzeichnet** ist, wobei die Oligonukleotid-Sonden in einem besonderen Satz für die Hybridisierung mit einer Nukleotid-Zielsequenz, die sich von anderen Nukleotidsequenzen in dem Prüfgut durch eine oder mehrere einzelne Basenveränderungen, Baseneinfügungen, Basendeletionen oder -translokationen unterscheidet, sowie für die Ligation miteinander geeignet sind, wenn sie nebeneinander auf der Nukleotid-Zielsequenz hybridisiert werden, jedoch eine Fehlpaarung aufweisen, die eine derartige Ligation stört, wenn sie mit irgendeiner anderen, im Prüfgut vorhandenen Nukleotidsequenz hybridisiert werden;
Bereitstellen einer thermostabilen Ligase nach Anspruch 1;
Vermischen des Prüfgutes, eines oder mehrerer Oligonukleotid-Sondensätze sowie der thermostabilen Ligase, um ein Reaktionsgemisch für den Nachweis von Ligase zu bilden;
Aussetzen des Reaktionsgemisches für den Ligasenachweis einem oder mehreren Reaktionszyklen für den Ligasenachweis, die eine Denaturierungsbehandlung, bei der alle hybridisierten Oligonukleotide von der Nukleotid-Zielsequenz getrennt werden, und eine Hybridisierungsbehandlung umfassen, bei der die Oligonukleotid-Sondensätze auf benachbarten Positionen in einer basenspezifischen Art und Weise mit ihren entsprechenden Nukleotid-Zielsequenzen hybridisieren, falls sie in dem Prüfgut vorhanden sind, und sich miteinander verknüpfen, um eine Ligationsproduktsequenz zu bilden, die zielspezifische Abschnitte enthält, die gemeinsam mit den Ligationsproduktsequenzen für jeden Satz verbunden sind, der von anderen Nukleinsäuren in dem Reaktionsgemisch für den Ligasenachweis unterscheidbar ist, bei dem die Oligonukleotid-Sondensätze mit einer Nukleotidsequenz (außer ihren jeweiligen Nukleotid-Zielsequenzen) im Prüfgut hybridisieren können, sich jedoch wegen des Vorhandenseins einer oder mehrerer Fehlpaarungen nicht miteinander verbinden und sich während der Denaturierungsbehandlung einzeln trennen; und
Feststellen des Vorhandenseins von Ligationsproduktsequenzen, die als Ergebnis der im Prüfgut vorhandenen Nukleotid-Zielsequenz entstanden sind.

9. Verfahren nach Anspruch 8, das weiterhin folgenden Verfahrensschritt umfasst: Amplifizieren der im Prüfgut vorhandenen Nukleotid-Zielsequenz vor dem Vermischen.

10. Verfahren nach Anspruch 9, bei dem das Amplifizieren durch eine Polymerasekettenreaktion ausgeführt wird.

11. Verfahren zum Nachweis einer doppelsträngigen Ziel-Nukleinsäure im Prüfgut, die aus ersten und zweiten komplementären Nukleotid-Zielsequenzen gebildet wird, welche sich von anderen Nukleotidsequenzen durch eine oder mehrere einzelne Basenveränderungen, Baseneinfügungen, Basendeletionen oder -translokationen unterscheiden, wobei das Verfahren folgende Schritte umfasst:
Bereitstellen von Prüfgut, das potentiell eine doppelsträngige Ziel-Nukleinsäure enthält, die aus ersten und zweiten komplementären Nukleotidsequenzen gebildet wird, welche sich von anderen Nukleotidsequenzen im Prüfgut durch eine oder mehrere einzelne Basenveränderungen, Baseneinfügungen, Basendeletionen oder -translokationen unterscheiden;
Bereitstellen eines ersten Oligonukleotid-Sondensatzes, der durch (a) eine erste Oligonukleotid-Sonde mit einem zielspezifischen Abschnitt und durch (b) eine zweite Oligonukleotid-Sonde mit einem zielspezifischen Abschnitt **gekennzeichnet** ist, wobei die Oligonukleotid-Sonden im ersten Satz komplementär zur ersten Nukleotid-Zielsequenz gehören, die sich von anderen Nukleotidsequenzen im Prüfgut durch eine oder mehrere einzelne Basenveränderungen, Baseneinfügungen, Basendeletionen oder -translokationen unterscheidet, und zur Ligation miteinander geeignet sind, wenn sie nebeneinander auf der ersten Nukleotid-Zielsequenz hybridisiert werden, jedoch eine bei einer derartigen Ligation störende Fehlpaarung aufweisen, wenn sie mit irgendeiner anderen, im Prüfgut vorhandenen Nukleotidsequenz hybridisiert werden;
Bereitstellen eines zweiten Oligonukleotid-Sondensatzes, der durch (a) eine dritte Oligonukleotid-Sonde mit einem zielspezifischen Abschnitt und durch (b) eine vierte Oligonukleotid-Sonde mit einem zielspezifischen Abschnitt **gekennzeichnet** ist, wobei die Oligonukleotid-Sonden im zweiten Satz komplementär zur zweiten Nukleotid-Zielsequenz gehören, die sich von anderen Nukleotidsequenzen im Prüfgut durch eine oder mehrere Basenveränderungen, Baseneinfügungen, Basendeletionen oder -translokationen unterscheidet, und zur Ligation miteinander geeignet sind, wenn sie nebeneinander auf der zweiten Nukleotid-Zielsequenz hybridisiert werden, jedoch eine bei einer derartigen Ligation störende Fehlpaarung aufweisen, wenn sie mit irgendeiner anderen, im Prüfgut vorhandenen Nukleotidsequenz hybridisiert werden;
Bereitstellen einer thermostabilen Ligase nach Anspruch 1;
Vermischen des Prüfgutes, der ersten und zweiten Oligonukleotid-Sondensätze und der thermostabilen Ligase, um ein Ligase-Kettenreaktionsgemisch zu bilden;
Aussetzen des Ligase-Kettenreaktionsgemisches einem oder mehreren Ligase-Kettenreaktionszyklen, die eine Denaturierungsbehandlung, bei der alle hybridisierten Oligonukleotide von den Nukleotid-Zielsequenzen getrennt werden, und eine Hybridisierungsbehandlung umfassen, bei der die Oligonukleotid-Sondensätze auf benachbarten Positionen in einer basenspezifischen Art und Weise mit ihren jeweiligen Nukleotid-Zielsequenzen hybridisieren, falls sie im Prüfgut vorhanden sind, und sich miteinander verbinden, um eine Ligationsproduktsequenz zu bilden, die zielspezifische Abschnitte enthalten, die gemeinsam mit den Ligationsproduktsequenzen für jeden Satz verbunden sind, der von anderen Nukleinsäuren im Ligase-Kettenreaktionsgemisch unterscheidbar ist, wobei die Oligonukleotid-Sondensätze mit Nukleotidsequenzen (außer ihren jeweiligen Nukleotid-Zielsequenzen) im Prüfgut hybridisieren können, sich aber wegen des Vorhandenseins einer oder mehrerer Fehlpaarungen nicht miteinander verbinden und sich während der Denaturierungsbehandlung einzeln trennen; und
Feststellen des Vorhandenseins von Ligationsproduktsequenzen, die als Ergebnis der im Prüfgut vorhandenen Nukleotid-Zielsequenz entstanden sind.

## Revendications

1. Ligase thermostable isolée, ladite ligase thermostable ayant la séquence d'acides aminés de SEQ ID NO: 1.

2. Molécule d'ADN isolée codant pour une ligase thermostable ayant la séquence d'acides aminés de SEQ ID NO: 1.

3. Molécule d'ADN isolée selon la revendication 2, dans laquelle ladite molécule d'ADN a la séquence nucléotidique de SEQ ID NO: 2.

4. Vecteur d'expression d'ADN transduit avec la molécule d'ADN hétérologue selon la revendication 2.

5. Vecteur d'expression d'ADN transduit avec la molécule d'ADN hétérologue selon la revendication 3.

6. Cellule hôte transduite avec la molécule d'ADN hétérologue selon la revendication 2.

7. Cellule hôte transduite avec la molécule d'ADN hétérologue selon la revendication 3.

8. Procédé de détection, dans un échantillon, d'une séquence nucléotidique cible qui diffère d'autres séquences nucléotidiques présentes dans l'échantillon par une ou plusieurs modifications, insertions, délétions ou translocations de bases individuelles, ledit procédé comprenant les étapes consistant à :
obtenir un échantillon contenant potentiellement une séquence nucléotidique cible qui diffère d'autres séquences nucléotidiques présentes dans l'échantillon par une ou plusieurs modifications, insertions, délétions ou translocations de bases individuelles ;
obtenir un ou plusieurs ensembles d'amorces oligonucléotidiques, **caractérisés** chacun par (a) une première amorce oligonucléotidique ayant une partie spécifique cible et (b) une deuxième amorce oligonucléotidique ayant une partie spécifique à la cible, les amorces oligonucléotidiques d'un ensemble particulier étant adaptées pour s'hybrider à une séquence nucléotidique cible qui diffère d'autres séquences nucléotidiques présentes dans l'échantillon par une ou plusieurs modifications, insertions, délétions ou translocations de bases individuelles et pour se souder ensemble lorsqu'elles sont hybridées adjacentes les unes aux autres sur la séquence nucléotidique cible, mais présentant un mésappariement qui perturbe cette soudure lorsqu'elles sont hybridées à toute autre séquence nucléotidique présente dans l'échantillon ;
obtenir une ligase thermostable selon la revendication 1;
mélanger l'échantillon, lesdits un ou plusieurs ensembles d'amorces oligonucléotidiques et la ligase thermostable afin de former un mélange réactionnel de détection de ligase ;
soumettre le mélange réactionnel de détection de ligase à un ou plusieurs cycles de réaction de détection de ligase comprenant un traitement de dénaturation, dans lequel les éventuels oligonucléotides hybridés sont séparés de la séquence nucléotidique cible, et un traitement d'hybridation, dans lequel les ensembles d'amorces oligonucléotidiques s'hybrident dans des positions adjacentes de manière spécifique aux bases à leurs séquences nucléotidiques cibles respectives, si elles sont présentes dans l'échantillon, et se soudent les uns aux autres afin de former une séquence de produit de ligature contenant les parties spécifiques cibles reliées ensemble avec les séquences de produits de ligature pour chaque ensemble qui est différenciable des autres acides nucléiques présents dans le mélange réactionnel de détection de ligase, les ensembles d'amorces oligonucléotidiques pouvant s'hybrider à une séquence nucléotidique présente dans l'échantillon autre que leurs séquences nucléotidiques cibles respectives, mais ne se soudant pas les uns aux autres à cause de la présence d'un ou plusieurs mésappariements et se séparant individuellement au cours du traitement de dénaturation ; et
détecter la présence de séquences de produits de ligature produites par suite de la présence de la séquence nucléotidique cible dans l'échantillon.

9. Procédé selon la revendication 8, comprenant en plus :
l'amplification, avant ledit mélangeage, de la séquence nucléotidique cible présente dans l'échantillon.

10. Procédé selon la revendication 9, dans lequel ladite amplification se fait par une réaction en chaîne de la polymérase.

11. Procédé de détection, dans un échantillon, d'un acide nucléique double brin cible formé à partir de première et deuxième séquences nucléotidiques cibles complémentaires qui diffèrent des autres séquences nucléotidiques par une ou plusieurs modifications, insertions, délétions ou translocations de bases individuelles, ledit procédé comprenant les étapes consistant à :
obtenir un échantillon contenant potentiellement un acide nucléique double brin cible formé à partir de première et deuxième séquences nucléotidiques complémentaires qui diffèrent d'autres séquences nucléotidiques présentes dans l'échantillon par une ou plusieurs modifications, insertions, délétions ou translocations de bases individuelles ;
obtenir un premier ensemble d'amorces oligonucléotidiques, **caractérisé par** (a) une première amorce oligonucléotidique ayant une partie spécifique cible et (b) une deuxième amorce oligonucléotidique ayant une partie spécifique à la cible, les amorces oligonucléotidiques du premier ensemble étant complémentaires à la première séquence nucléotidique cible qui diffère d'autres séquences nucléotidiques présentes dans l'échantillon par une ou plusieurs modifications, insertions, délétions ou translocations de bases individuelles et étant adaptées pour se souder ensemble lorsqu'elles sont hybridées adjacentes les unes aux autres sur la première séquence nucléotidique cible, mais présentant un mésappariement qui perturbe cette soudure lorsqu'elles sont hybridées à toute autre séquence nucléotidique présente dans l'échantillon ;
obtenir un deuxième ensemble d'amorces oligonucléotidiques, **caractérisé par** (a) une troisième amorce oligonucléotidique ayant une partie spécifique cible et (b) une quatrième amorce oligonucléotidique ayant une partie spécifique à la cible, les amorces oligonucléotidiques du deuxième ensemble étant complémentaires à la deuxième séquence nucléotidique cible qui diffère d'autres séquences nucléotidiques présentes dans l'échantillon par une ou plusieurs modifications, insertions, délétions ou translocations de bases individuelles et étant adaptées pour se souder ensemble lorsqu'elles sont hybridées adjacentes les unes aux autres sur la deuxième séquence nucléotidique cible, mais présentant un mésappariement qui perturbe cette soudure lorsqu'elles sont hybridées à toute autre séquence nucléotidique présente dans l'échantillon ;
obtenir une ligase thermostable selon la revendication 1 ;
mélanger l'échantillon, les premier et deuxième ensembles d'amorces oligonucléotidiques et la ligase thermostable afin de former un mélange de réaction en chaîne de la ligase ;
soumettre le mélange de réaction en chaîne de la ligase à un ou plusieurs cycles de réaction en chaîne de la ligase comprenant un traitement de dénaturation, dans lequel les éventuels oligonucléotides hybridés sont séparés des séquences nucléotidiques cibles, et un traitement d'hybridation, dans lequel les ensembles d'amorces oligonucléotidiques s'hybrident dans des positions adjacentes de manière spécifique aux bases à leurs séquences nucléotidiques cibles respectives, si elles sont présentes dans l'échantillon, et se soudent les uns aux autres afin de former une séquence de produit de ligature contenant les parties spécifiques cibles reliées ensemble avec la séquence de produit de ligature pour chaque ensemble qui est différenciable des autres acides nucléiques dans le mélange de réaction en chaîne de la ligase, les ensembles d'amorces oligonucléotidiques pouvant s'hybrider à des séquences nucléotidiques présentes dans l'échantillon autres que leurs séquences nucléotidiques cibles respectives, mais ne se soudant pas les uns aux autres du fait de la présence d'un ou plusieurs mésappariements et se séparant individuellement au cours du traitement de dénaturation ; et
détecter la présence de séquences de produits de ligature produites par suite de la présence de la séquence nucléotidique cible dans l'échantillon.


### SEQUENCE LISTING

<110> Cornell Research Foundation, Inc.
<120> HIGH FIDELITY THERMOSTABLE LIGASE AND USES THEREOF
<130> 19603/2612
<140> PCT/US99/25437
   <141> 1999-10-29
<150> 60/106,461
   <151> 1998-10-30
<160> 20
<170> PatentIn Ver. 2.1
<210> 1
   <211> 674
   <212> PRT
   <213> Thermus sp.
<400> 1
   Met Thr Leu Glu Glu Ala Arg Arg Arg Val Asn Glu Leu Arg Asp Leu
     1               5                  10                  15
   Ile Arg Tyr His Asn Tyr Leu Tyr Tyr Val Leu Asp Ala Pro Glu Ile
                20                  25                  30
   Ser Asp Ala Glu Tyr Asp Arg Leu Leu Arg Glu Leu Lys Glu Leu Glu
            35                  40                  45
   Glu Arg Phe Pro Glu Leu Lys Ser Pro Asp Ser Pro Thr Glu Gln Val
        50                  55                  60
   Gly Ala Arg Pro Leu Glu Ala Thr Phe Arg Pro Val Arg His Pro Thr
    65                  70                  75                  80
   Arg Met Tyr Ser Leu Asp Asn Ala Phe Ser Leu Asp Glu Val Arg Ala
                    85                  90                  95
   Phe Glu Glu Arg Ile Glu Arg Ala Leu Gly Arg Lys Gly Pro Phe Leu
               100                 105                 110
   Tyr Thr Val Glu Arg Lys Val Asp Gly Leu Ser Val Asn Leu Tyr Tyr
           115                 120                 125
   Glu Glu Gly Ile Leu Val Phe Gly Ala Thr Arg Gly Asp Gly Glu Thr
   130 135 140
   Gly Glu Glu Val Thr Gln Asn Leu Leu Thr Ile Pro Thr Ile Pro Arg
   145 150 155 160
   Arg Leu Thr Gly Val Pro Asp Arg Leu Glu Val Arg Gly Glu Val Tyr
   165 170 175
   Met Pro Ile Glu Ala Phe Leu Arg Leu Asn Gln Glu Leu Glu Glu Ala
   180 185 190
   Gly Glu Arg Ile Phe Lys Asn Pro Arg Asn Ala Ala Ala Gly Ser Leu
   195 200 205
   Arg Gln Lys Asp Pro Arg Val Thr Ala Arg Arg Gly Leu Arg Ala Thr
   210 215 220
   Phe Tyr Ala Leu Gly Leu Gly Leu Glu Glu Thr Gly Leu Lys Ser Gln
   225 230 235 240
   His Asp Leu Leu Leu Trp Leu Arg Glu Arg Gly Phe Pro Val Glu His
   245 250 255
   Gly Phe Thr Arg Ala Leu Gly Ala Glu Gly Val Glu Glu Val Tyr Gln
   260 265 270
   Ala Trp Leu Lys Glu Arg Arg Lys Leu Pro Phe Glu Ala Asp Gly Val
   275 280 285
   Val Val Lys Leu Asp Asp Leu Ala Leu Trp Arg Glu Leu Gly Tyr Thr
   290 295 300
   Ala Arg Thr Pro Arg Phe Ala Leu Ala Tyr Lys Phe Pro Ala Glu Glu
   305 310 315 320
   Lys Glu Thr Arg Leu Leu Ser Val Ala Phe Gln Val Gly Arg Thr Gly
   325 330 335
   Arg Ile Thr Pro Val Gly Val Leu Glu Pro Val Phe Ile Glu Gly Ser
   340 345 350
   Glu Val Ser Arg Val Thr Leu His Asn Glu Ser Phe Ile Glu Glu Leu
   355 360 365
   Asp Val Arg Ile Gly Asp Trp Val Leu Val His Lys Ala Gly Gly Val
   370 375 380
   Ile Pro Glu Val Leu Arg Val Leu Lys Glu Arg Arg Thr Gly Glu Glu
   385 390 395 400
   Lys Pro Ile Ile Trp Pro Glu Asn Cys Pro Glu Cys Gly His Ala Leu
   405 410 415
   Ile Lys Glu Gly Lys Val His Arg Cys Pro Asn Pro Leu Cys Pro Ala
   420 425 430
   Lys Arg Phe Glu Ala Ile Arg His Tyr Ala Ser Arg Lys Ala Met Asp
   435 440 445
   Ile Gln Gly Leu Gly Glu Lys Leu Ile Glu Lys Leu Leu Glu Lys Gly
   450 455 460
   Leu Val Arg Asp Val Ala Asp Leu Tyr Arg Leu Lys Lys Glu Asp Leu
   465 470 475 480
   Val Asn Leu Glu Arg Met Gly Glu Lys Ser Ala Glu Asn Leu Leu Arg
   485 490 495
   Gln Ile Glu Glu Ser Lys Gly Arg Gly Leu Glu Arg Leu Leu Tyr Ala
   500 505 510
   Leu Gly Leu Pro Gly Val Gly Glu Val Leu Ala Arg Asn Leu Ala Leu
   515 520 525
   Arg Phe Gly His Met Asp Arg Leu Leu Glu Ala Gly Leu Glu Asp Leu
   530 535 540
   Leu Glu Val Glu Gly Val Gly Glu Leu Thr Ala Arg Ala Ile Leu Asn
   545 550 555 560
   Thr Leu Lys Asp Pro Glu Phe Arg Asp Leu Val Arg Arg Leu Lys Glu
   565 570 575
   Ala Gly Val Glu Met Glu Ala Lys Glu Arg Glu Gly Glu Ala Leu Lys
   580 585 590
   Gly Leu Thr Phe Val Ile Thr Gly Glu Leu Ser Arg Pro Arg Glu Glu
   595 600 605
   Val Lys Ala Leu Leu Arg Arg Leu Gly Ala Lys Val Thr Asp Ser Val
   610 615 620
   Ser Arg Lys Thr Ser Phe Leu Val Val Gly Glu Asn Pro Gly Ser Lys
   625 630 635 640
   Leu Glu Lys Ala Arg Ala Leu Gly Val Pro Thr Leu Ser Glu Glu Glu
                   645                 650                 655
   Leu Tyr Arg Leu Ile Glu Glu Arg Thr Gly Lys Asp Pro Arg Ala Leu
               660                 665                 670
   Thr Ala
<210> 2
   <211> 2025
   <212> DNA
   <213> Thermus sp.
<400> 2
   atgaccctag aggaggcccg caggcgcgtc aacgaactca gggacctgat ccgttaccac 60
   aactacctct attacgtctt ggacgccccc gagatctccg acgccgagta cgaccggctc 120
   cttagggagc ttaaggagct ggaggagcgc tttcccgagc tcaaaagccc cgactccccc 180
   acggaacagg tgggggcgag gcctctggag gccaccttcc gcccggtgcg ccaccccacc 240
   cgcatgtact ccctggacaa cgccttttcc ttggacgagg tgagggcctt tgaggagcgc 300
   atagagcggg ccctggggcg gaaggggccc ttcctctaca ccgtggagcg caaggtggac 360
   ggtctttccg tgaacctcta ctacgaggag ggcatcctcg tctttggggc cacccggggc 420
   gacggggaga ccggggagga ggtgacccag aacctcctca ccatccccac cattccccgc 480
   cgcctcacgg gcgttccgga ccgcctcgag gtccggggcg aggtctacat gcccatagag 540
   gccttcctca ggctcaacca ggagctggag gaggcggggg agcgcatctt caaaaacccc 600
   aggaacgccg ccgccgggtc cttgcggcag aaagacccca gggtcacggc caggcggggc 660
   ctgagggcca ccttttacgc cctggggctg ggcctggagg aaaccgggtt aaaaagccag 720
   cacgaccttc tcctatggct aagagagcgg ggctttcccg tggagcacgg ctttacccgg 780
   gccctggggg cggagggggt ggaggaggtc taccaggcct ggctcaagga gaggcggaag 840
   cttccctttg aggccgacgg ggtggtggtc aagctggacg acctcgccct ctggcgggag 900
   ctggggtaca ccgcccgcac cccccgcttc gccctcgcct acaagttccc ggccgaggag 960
   aaggagaccc gcctcctctc cgtggccttc caggtggggc ggaccgggcg catcaccccc 1020
   gtgggcgttc tggagcccgt cttcatagag ggcagcgagg tgagccgggt caccctccac 1080
   aacgagagct tcattgagga gctggacgtg cgcatcggcg actgggtgct ggtccacaag 1140
   gcgggcgggg tgattcccga ggtgctgagg gtcctgaaag agcgccgcac cggggaggag 1200
   aagcccatca tctggcccga gaactgcccc gagtgcggcc acgccctcat caaggagggg 1260
   aaggtccacc gctgccccaa ccccttgtgc cccgccaagc gctttgaggc catccgccac 1320
   tacgcctccc gcaaggccat ggacatccag ggcctggggg agaagctcat agaaaagctt 1380
   ctggaaaagg gcctggtccg ggacgtggcc gacctctacc gcctgaagaa ggaggacctg 1440
   gtgaacctgg agcgcatggg ggagaagagc gcagagaacc tcctccgcca gatagaggag 1500
   agcaagggcc gcggcctgga gcgcctcctt tacgccctgg gccttcccgg ggtgggggag 1560
   gtgctggccc ggaacctggc cctccgcttc ggccacatgg accgccttct ggaggcgggc 1620
   ctcgaggacc tcctggaggt ggagggggtg ggcgagctca ccgcccgggc catcctgaat 1680
   accctaaagg acccggagtt ccgggacctg gtgcgccgcc tgaaggaggc cggggtggag 1740
   atggaggcca aagagcggga gggcgaggcc ttgaaggggc tcaccttcgt catcaccggg 1800
   gagctttccc ggccccggga ggaggtgaag gccctcctta ggcggcttgg ggccaaggtg 1860
   acggactcgg tgagccgcaa gacgagcttc ctggtggtgg gggagaaccc ggggagcaag 1920
   ctggaaaagg cccgcgcctt gggggtcccc accctgagcg aggaggagct ctaccgcctc 1980
   attgaggaga ggacgggcaa ggacccaagg gccctcacgg cctag 2025
<210> 3
   <211> 20
   <212> DNA
   <213> Artificial Sequence
<220>
   <223> Description of Artificial Sequence: probe or primer
<400> 3
   atcwscgacg csgartayga 20
<210> 4
   <211> 7
   <212> PRT
   <213> Artificial Sequence
<220>
   <223> Description of Artificial Sequence: probe or primer
<400> 4
   Ile Ser Asp Ala Glu Tyr Asp
   1 5
<210> 5
   <211> 20
   <212> DNA
   <213> Artificial Sequence
<220>
   <223> Description of Artificial Sequence: probe or primer
<400> 5
   ccsgtscksc csacytgraa 20
<210> 6
   <211> 20
   <212> DNA
   <213> Artificial Sequence
<220>
   <223> Description of Artificial Sequence: probe or primer
<400> 6
   gccttctcva ryttgswscc 20
<210> 7
   <211> 7
   <212> PRT
   <213> Artificial Sequence
<220>
   <223> Description of Artificial Sequence: probe or primer
<400> 7
   Phe Gln Val Gly Arg Thr Gly
   1               5
<210> 8
   <211> 7
   <212> PRT
   <213> Artificial Sequence
<220>
   <223> Description of Artificial Sequence: probe or primer
<400> 8
   Gly Ser Lys Leu Glu Lys Ala
   1               5
<210> 9
   <211> 30
   <212> DNA
   <213> Artificial Sequence
<220>
   <223> Description of Artificial Sequence: probe or primer
<400> 9
   gcgatttcat atgaccctag aggaggcccg 30
<210> 10
   <211> 29
   <212> DNA
   <213> Artificial Sequence
<220>
   <223> Description of Artificial Sequence: probe or primer
<400> 10
   gcgggatccg aggccttgga gaagctctt 29
<210> 11
   <211> 33
   <212> DNA
   <213> Artificial Sequence
<220>
   <223> Description of Artificial Sequence: probe or primer
<400> 11
   aaaaccctgt tccagcgtct gcggtgttgc gtc 33
<210> 12
   <211> 30
   <212> DNA
   <213> Artificial Sequence
<220>
   <223> Description of Artificial Sequence: probe or primer
<400> 12
   agttgtcata gtttgatcct ctagtctggg 30
<210> 13
   <211> 29
   <212> DNA
   <213> Artificial Sequence
<220>
   <223> Description of Artificial Sequence: probe or primer
<400> 13
   ccctgttcca gcgtctgcgg tgttgcgtt 29
<210> 14
   <211> 59
   <212> DNA
   <213> Artificial Sequence
<220>
   <223> Description of Artificial Sequence: probe or primer
<400> 14
   gggacaaggt cgcagacgcc acaacgcagt caacagtatc aaactaggag atcagaccc 59
<210> 15
   <211> 184
   <212> PRT
   <213> Thermus aquaticus
<400> 15
   Tyr Thr Val Glu His Lys Val Asp Gly Leu Ser Val Asn Leu Tyr Tyr
   1 5 10 15
   Glu Xaa Xaa Xaa Xaa Xaa Xaa Xaa Xaa Xaa Xaa Xaa Xaa Xaa Xaa Xaa
   20 25 30
   Xaa Xaa Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Xaa Xaa Xaa Xaa Xaa Xaa Xaa Xaa Xaa Xaa Xaa Xaa Xaa Xaa
   100 105 110
   Xaa Xaa Xaa Xaa Xaa Xaa Xaa Xaa Leu Glu Glu Thr Gly Xaa Xaa Xaa
   115 120 125
   Xaa Xaa Xaa Xaa Xaa Xaa Xaa Xaa Xaa Xaa Xaa Xaa Xaa Xaa Xaa Xaa
   130 135 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Pro Phe Glu Ala
   165 170 175
   Asp Gly Val Val Val Lys Leu Asp
   180
<210> 16
   <211> 187
   <212> PRT
   <213> Thermus flavus
<400> 16
   Tyr Thr Val Glu His Lys Val Asp Gly Leu Ser Val Asn Leu Tyr Tyr
   1 5 10 15
   Glu Xaa Xaa Xaa Xaa Xaa Xaa Xaa Xaa Xaa Xaa Xaa Xaa Xaa Xaa Xaa
   20 25 30
   Xaa Xaa Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Xaa Xaa Xaa Xaa Xaa Xaa Xaa Xaa Xaa Xaa Xaa Xaa Xaa Xaa
   100 105 110
   Xaa Xaa Xaa Xaa Xaa Xaa Xaa Xaa Leu Glu Glu Val Glu Arg Glu Gly
   115 120 125
   Xaa Xaa Xaa Xaa Xaa Xaa Xaa Xaa Xaa Xaa Xaa Xaa Xaa Xaa Xaa Xaa
   130 135 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Xaa Xaa Xaa Pro
   165 170 175
   Phe Glu Ala Asp Gly Val Val Val Lys Leu Asp
   180 185
<210> 17
   <211> 184
   <212> PRT
   <213> Thermus filiformis
<400> 17
   Tyr Thr Val Glu His Lys Val Asp Gly Leu Ser Val Asn Leu Tyr Tyr
   1               5                   10                  15
   Glu Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Xaa Xaa Xaa Xaa Leu Glu Glu Ser Gly Xaa Xaa Xaa
           115                 120                 125
   Xaa Xaa Xaa Xaa Xaa Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145                 150                 155                 160
   Xaa Xaa Xaa Xaa Xaa Xaa Xaa Xaa Xaa Xaa Xaa Xaa Pro Phe Glu Ala
                   165                 170                 175
   Asp Gly Val Val Val Lys Met Asp
               180
<210> 18
   <211> 184
   <212> PRT
   <213> Thermus filiformis
<400> 18
   Tyr Thr Val Glu His Lys Val Asp Gly Leu Ser Val Asn Leu Tyr Tyr
   1 5 10 15
   Glu Xaa Xaa Xaa Xaa Xaa Xaa Xaa Xaa Xaa Xaa Xaa Xaa Xaa Xaa Xaa
   20 25 30
   Xaa Xaa Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Xaa Xaa Xaa Xaa Xaa Xaa Xaa Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Xaa Xaa Xaa Xaa Xaa Xaa Xaa Xaa Xaa Xaa Xaa Xaa Xaa Xaa
   100 105 110
   Xaa Xaa Xaa Xaa Xaa Xaa Xaa Xaa Leu Glu Glu Ser Gly Xaa Xaa Xaa
   115 120 125
   Xaa Xaa Xaa Xaa Xaa Xaa Xaa Xaa Xaa Xaa Xaa Xaa Xaa Xaa Xaa Xaa
   130 135 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Pro Phe Glu Ala
   165 170 175
   Asp Gly Val Val Val Lys Leu Asp
   180
<210> 19
   <211> 184
   <212> PRT
   <213> Thermus sp.
<400> 19
   Tyr Thr Val Glu His Lys Val Asp Gly Leu Ser Val Asn Leu Tyr Tyr
   1               5                   10                  15
   Glu Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Xaa Xaa Xaa Xaa Leu Glu Glu Ser Gly Xaa Xaa Xaa
           115                 120                 125
   Xaa Xaa Xaa Xaa Xaa Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145                 150                 155                 160
   Xaa Xaa Xaa Xaa Xaa Xaa Xaa Xaa Xaa Xaa Xaa Xaa Pro Phe Glu Ala
                   165                 170                 175
   Asp Gly Val Val Val Lys Leu Asp
               180
<210> 20
   <211> 184
   <212> PRT
   <213> Thermus sp.
<400> 20
   Tyr Thr Val Glu His Lys Val Asp Gly Leu Ser Val Asn Leu Tyr Tyr
    1               5                  10                  15
   Glu Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa Xaa Xaa Xaa Xaa Xaa Xaa Xaa Xaa Xaa Xaa Xaa Xaa Xaa Xaa Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Xaa Xaa
               100                 105                 110
   Xaa Xaa Xaa Xaa Xaa Xaa Xaa Xaa Leu Glu Glu Ser Gly Xaa Xaa Xaa
           115                 120                 125
   Xaa Xaa Xaa Xaa Xaa Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145                 150                 155                 160
   Xaa Xaa Xaa Xaa Xaa Xaa Xaa Xaa Xaa Xaa Xaa Xaa Pro Phe Glu Ala
                   165                 170                 175
   Asp Gly Val Val Val Lys Leu Asp
               180